# EUROPÄISCHE PATENTSCHRIFT

(11) **EP 1 135 387 B1**
(45) Veröffentlichungstag und Bekanntmachung des Hinweises auf die Patenterteilung: **06.11.2002**
(21) Anmeldenummer: 99961051.2
(22) Anmeldetag: 02.12.1999
(51) Int. Cl.: C07D 413/10, C07D 231/20, A01N 43/74, A01N 43/50

(54) **3-(HETEROCYCLYL)-BENZOYLPYRAZOL-DERIVATE**
3-(HETEROCYCLYL)-BENZOYLPYRAZOLE-DERIVATIVES
DERIVES DE 3-(HETEROCYCLYL)-BENZOYLPYRAZOLE

(30) Priorität: 04.12.1998 DE 19855850; 06.08.1999 DE 19936705
(43) Veröffentlichungstag der Anmeldung: 26.09.2001
(73) Patentinhaber: BASF AKTIENGESELLSCHAFT, 67056 Ludwigshafen (DE)
(72) Erfinder: NEIDLEIN, UlF, D-68165 Mannheim (DE); GÖTZ, Norbert, D-67547 Worms (DE); MISSLITZ, Ulf, D-67433 Neustadt (DE); GÖTZ, Roland, D-68809 Neulussheim (DE); BAUMANN, Ernst, D-67373 Dudenhofen (DE); VON DEYN, Wolfgang, D-67435 Neustadt (DE); KUDIS, Steffen, D-68167 Mannheim (DE); LANGEMANN, Klaus, D-67551 Worms (DE); MAYER, Guido, D-67433 Neustadt (DE); WITSCHEL, Matthias, D-67061 Ludwigshafen (DE); OTTEN, Martina, D-67069 Ludwigshafen (DE); WESTPHALEN, Karl-Otto, D-67346 Speyer (DE); WALTER, Helmut, D-67283 Obrigheim (DE)
(86) Internationale Anmeldenummer: EP9909412
(87) Internationale Veröffentlichungsnummer: WO00034272

(56) Entgegenhaltungen:
- WO-A-96/26206
- WO-A-98/31681

## Beschreibung

Die vorliegende Erfindung betrifft 3- (Heterocyclyl) -benzoylpyrazol-Derivate der Formel I 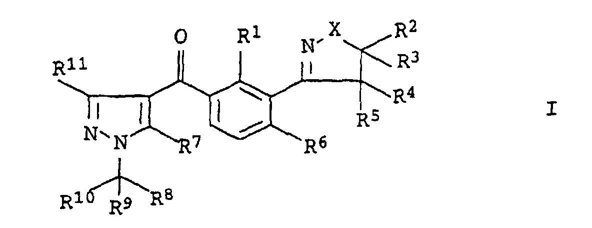 in der die Variablen folgende Bedeutung haben:
- X: O, NH oder N(C₁-C₆-Alkyl);
- R¹: Nitro, Halogen, C₁-C₄-Alkoxy, C₁-C₄-Halogenalkoxy, C₁-C₄-Alkylthio, C₁-C₄-Halogenalkylthio, C₁-C₄-Alkylsulfonyl oder C₁-C₄-Halogenalkylsulfonyl;
- R² ,R³ ,R⁴ ,R⁵: Wasserstoff, C₁-C₄-Alkyl oder C₁-C₄-Halogenalkyl;
- R⁶: Halogen, Nitro, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfonyl oder C₁-C₄-Halogenalkylsulfonyl;
- R⁷: Hydroxy, C₁-C₆-Alkoxy, C₃-C₆-Alkenyloxy, C₁-C₆-Alkylsulfonyloxy, C₁-C₆-Alkylcarbonyloxy, C₁-C₄-(Alkylthio) carbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy oder C₁-C₄-Halogenalkoxy;
- R⁸ ,R⁹: C₁-C₄-Alkyl;
- R¹⁰: Wasserstoff oder C₁-C₄-Alkyl;
wobei die Anzahl der Kohlenstoffatome der Reste R⁸, R⁹ und R¹⁰ zusammen maximal 7 beträgt,
- R¹¹: Wasserstoff oder C₁-C₄-Alkyl;
sowie deren landwirtschaftlich brauchbaren Salze.

Außerdem betrifft die Erfindung Verfahren zur Herstellung von Verbindungen der Formel I, Mittel welche diese enthalten sowie die Verwendung dieser Derivate oder diese enthaltende Mittel zur Schadpflanzenbekämpfung.

Aus der Literatur, beispielsweise aus WO 96/26206 und WO 98/31681 sind Pyrazol-4-yl-benzoylderivate bekannt.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die 3-(Heterocyclyl)-benzoylpyrazol-Derivate der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren Phosphoniumionen, Sulfoniumionen, vorzugsweise Tri (C₁-C₄-alkyl) sulfonium und Sulfoxoniumionen, vorzugsweise Tri (C₁-C₄-alkyl) sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R¹¹ oder als Reste an Phenylringen genannten organischen Molekülteile stellen Sammelbegriffe für individuelle Aufzählungen der einzelnen Gruppenmitglieder dar. Sämtliche Kohlenwasserstoffketten, also alle Alkyl-, Alkylcarbonyl-, Halogenalkyl-, Alkoxy-, Halogenalkoxy-, Alkylcarbonyloxy-, (Alkylthio)carbonyloxy-, Alkylsulfonyloxy-, Alkylthio-, Halogenalkylthio-, Alkylsulfonyl-, Halogenalkylsulfonyl-, Alkenyl- und Alkenyloxy-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl, sowie die Alkylteile von C₁-C₄-Alkylcarbonyl und C₁-C₄-Alkylcarbonyloxy: z.B. Methyl, Ethyl, Propyl, 1-Methylethyl, Butyl, 1-Methylpropyl, 2-Methylpropyl und 1,1-Dimethylethyl;
- C₁-C₆-Alkyl, sowie die Alkylteile von C₁-C₆-Alkylcarbonyl und C₁-C₆-Alkylcarbonyloxy: C₁-C₄-Alkyl, wie voranstehend genannt, sowie z.B. Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Haiogenalkyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, Brommethyl, Iodmethyl, 1-Fluorethyl, 1-Chlorethyl, 1-Bromethyl, 1-Iodethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₄-Alkoxy: z.B. Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy: C₁-C₄-Alkoxy wie voranstehend genannt, sowie z.B. Pentoxy, 1-Methylbuboxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy,1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-l-methylpropoxy und 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₄-Alkylthio sowie die Alkylthioteile von C₁-C₄-(Alkylthio)carbonyloxy: z.B. Methylthio, Ethylthio, Propylthio, 1-Methylethylthio, Butylthio, 1-Methylpropylthio, 2-Methylpropylthio und 1,1-Dimethylethylthio;
- C₁-C₄-Halogenalkylthio: einen C₁-C₄-Alkylthiorest wie voranstehend genannt, der partiell oder vollständig durch Fluor, Chlor, Brom und/oder Iod substituiert ist, also z.B. Fluormethylthio, Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio und Nonafluorbutylthio;
- C₁-C₄-Alkylsulfonyl (C₁-C₄-Alkyl-S(=O)₂-), sowie die Alkylsulfonylteile von C₁-C₄-Alkylsulfonyloxy: z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
- C₁-C₆-Alkylsulfonyl, sowie die Alkylsulfonylteile von C₁-C₆-Alkylsulfonyloxy: einen C₁-C₄-Alkylsulfonylrest wie voranstehend genannt, sowie z.B. Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1 -Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 2,2,2-Trimethylpropylsulfonyl, 1-Ethyl-1-methylpropylsulfonyl und 1-Ethyl-2-methylpropylsulfonyl;
- C₁-C₄-Halogenalkylsulfonyl: einen C₁-C₄-Alkylsulfonylrest wie voranstehend genannt, der partiell oder vollständig durch Fluor, Chlor, Brom und/oder Iod substituiert ist, also z.B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl und Nonafluorbutylsulfonyl;
- C₃-C₆-Alkenyloxy: z.B. Prop-1-en-1-yloxy, Prop-2-en-1-yloxy, 1-Methylethenyloxy, Buten-1-yloxy, Buten-2-yloxy, Buten-3-yloxy, 1-Methyl-prop-1-en-1-yloxy, 2-Methyl-prop-1-en-1-yloxy, 1-Methyl-prop-2-en-1-yloxy, 2-Methyl-prop-2-en-1-yloxy, Penten-1-yloxy, Penten-2-yloxy, Penten-3-yloxy, Penten-4-yloxy, 1-Methyl-but-1-en-1-yloxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methyl-but-2-en-1-yloxy, 1-Methylbut-3-en-1-yloxy, 2-Methyl-but-3-en-1-yloxy, 3-Methylbut-3-en-1-yloxy, 1,1-Dimethyl-prop-2-en-1-yloxy, 1,2-Dimethyl-prop-1-en-l-yloxy, 1,2-Dimethyl-prop-2-en-1-yloxy, 1-Ethyl-prop-1-en-2-yloxy, 1-Ethyl-prop-2-en-1-yloxy, Hex-1-en-1-yloxy, Hex-2-en-1-yloxy, Hex-3-en-1-yloxy, Hex-4-en-1-yloxy, Hex-5-en-1-yloxy, 1-Methyl-pent-1-en-1-yloxy, 2-Methyl-pent-1-en-1-yloxy, 3-Methyl-pent-1-en-1-yloxy, 4-Methyl-pent-1-en-1-yloxy, 1-Methyl-pent-2-en-1-yloxy, 2-Methyl-pent-2-en-1-yloxy, 3-Methyl-pent-2-en-1-yloxy, 4-Methylpent-2-en-1-yloxy, 1-Methyl-pent-3-en-1-yloxy, 2-Methylpent-3-en-1-yloxy, 3-Methyl-pent-3-en-1-yloxy, 4-Methylpent-3-en-1-yloxy, 1-Methyl-pent-4-en-1-yloxy, 2-Methylpent-4-en-1-yloxy, 3-Methyl-pent-4-en-1-yloxy, 4-Methylpent-4-en-1-yloxy, 1,1-Dimethyl-but-2-en-1-yloxy, 1,1-Dimethyl-but-3-en-1-yloxy, 1,2-Dimethyl-but-1-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 2,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, 1-Ethyl-but-2-en-1-yloxy, 1-Ethyl-but-3-en-1-yloxy, 2-Ethylbut-1-en-1-yloxy, 2-Ethyl-but-2-en-1-yloxy, 2-Ethylbut-3-en-1-yloxy, 1,1,2-Trimethyl-prop-2-en-1-yloxy, 1-Ethyl-1-methyl-prop-2-en-1-yloxy, 1-Ethyl-2-methylprop-1-en-1-yloxy und 1-Ethyl-2-methyl-prop-2-en-1yloxy;
- C₃-C₆-Alkenyl: Prop-1-en-1 Prop-2-en-1-yl, 1-Methylethenyl, Buten-1-yl, Buten-2-yl, Buten-3-yl, 1-Methylprop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-1-yl, 2-Methylbut-1-en-1-yl, 3-Methyl-but-1-en-1-yl, 1-Methylbut-2-en-1-yl, 2-Methyl-but-2-en-1-yl, 3-Methylbut-2-en-1-yl, 1-Methyl-but-3-en-1-yl, 2-Methylbut-3-en-1-yl, 3-Methyl-but-3-en-1-yl, 1,1-Dimethylprop-2-en-1-yl, 1,2-Dimethyl-prop-1-en-1-yl, 1,2-Dimethylprop-2-en-1-yl, 1-Ethyl-prop-1-en-2-yl, 1-Ethylprop-2-en-1-yl, Hex-1-en-1- Hex-2-en-1-y Hex-3-en-1 Hex-4-en-1 Hex-5-en-1"yl, 1-Methyl-pent-1-en-1-yl, 2-Methyl-pent-1-en-1-yl, 3-Methyl-pent-1-en-1-yl, 4-Methylpent-1-en-1-yl, 1-Methyl-pent-2-en-1-yl, 2-Methylpent-2-en-1-yl, 3-Methyl-pent-2-en-1-yl, 4-Methylpent-2-en-1-yl, 1-Methyl-pent-3-en-1-yl, 2-Methylpent-3-en-1-yl, 3-Methyl-pent-3-en-1-yl, 4-Methylpent-3-en-1-yl, 1-Methyl-pent-4-en-1-yl, 2-Methylpent-4-en-1-yl, 3-Methyl-pent-4-en-1-yl, 4-Methylpent-4-en-1-yl, 1,1-Dimethyl-but-2-en-1-yl, 1,1-Dimethylbut-3-en-1-yl, 1,2-Dimethyl-but-1-en-1-yl, 1,2-Dimethylbut-2-en-1-yl, 1,2-Dimethyl-but-3-en-1-yl, 1,3-Dimethylbut-1-en-1-yl, 1,3-Dimethyl-but-2-en-1-yl, 1,3-Dimethyl-but-3-en-1-yl, 2,2-Dimethyl-but-3-en-1-yl, 2,3-Dimethylbut-1-en-1-yl, 2,3-Dimethyl-but-2-en-1-yl, 2,3-Dimethylbut-3-en-1-yl, 3,3-Dimethyl-but-1-en-1-yl, 3,3-Dimethylbut-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethylbut-2-en-1-yl, 2-Ethyl-but-3-en-1-yl, 1,1,2-Trimethylprop-2-en-1-yl, 1-Ethyl-1-methyl-prop-2-en-1-yl, 1-Ethyl-2-methyl-prop-1-en-1-yl und 1-Ethyl-2-methylprop-2-en-1-yl;

Die Phenylringe sind vorzugsweise unsubstituiert oder tragen ein bis drei Halogenatome und/oder eine Nitrogruppe, eine Cyano-gruppe, eine oder zwei Methyl-, Trifluormethyl-, Methoxy- oder Trifluormethoxygruppen.

Betont seien diejenigen Verbindungen der Formel I, wobei
- R⁷: für Hydroxy, C₁-C₆-Alkoxy, C₃-C₆-Alkenyloxy, C₁-C₆-Alkylsulfonyloxy, C₁-C₆-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy oder C₁-C₄-Halogenalkoxy;
steht.
- Bevorzugt sind die 3-(Heterocyclyl)-benzoylpyrazol-Derivate der Formel I, in der die Variablen folgende Bedeutung haben:
   - X: O;
   - R¹: Nitro, Halogen, C₁-C₄-Alkoxy oder C₁-C₄-Alkylthio; besonders bevorzugt Nitro, Halogen oder C₁-C₄-Alkoxy; insbesonders bevorzugt Halogen, wie Fluor, Chlor oder Brom, oder C₁-C₄-Alkoxy, wie Methoxy oder Ethoxy; insbesonderst bevorzugt Chlor oder Methoxy;
   - R² ,R³ ,R⁴ ,R⁵: Wasserstoff, C₁-C₄-Alkyl oder C₁-C₄-Halogenalkyl; besonders bevorzugt Wasserstoff, Methyl, Ethyl, Propyl, 1-Methylethyl, Fluormethyl oder Chlormethyl; insbesondere bevorzugt Wasserstoff, Methyl, Ethyl oder Chlormethyl;
   - R⁶: C₁-C₄-Alkylthio oder C₁-C₄-Alkylsulfonyl; besonders bevorzugt Methylthio, Ethylthio oder 1-Methyl-1-ethylthio, Methylsulfonyl, Ethylsulfonyl, 1-Methylethylsulfony oder Propylsulfonyl; insbesondere bevorzugt Methylsulfonyl, Ethylsulfonyl, 1-Methylethylsulfonyl oder Propylsulfonyl;
   - R⁷: Hydroxy, C₁-C₆-Alkoxy, C₃-C₆ -Alkenyloxy, C₁-C₆-Alkylsulfonyloxy, C₁-C₆-Alkylcarbonyloxy, C₁-C₆-(Alkylthio)carbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy; besonders bevorzugt Hydroxy, C₁-C₄-Alkoxy, C₃-C₆-Alkenyloxy, C₁-C₄-Alkylsulfonyloxy, C₁-C₄-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy;
   - R⁸ ,R⁹: C₁-C₄-Alkyl;
   besonders bevorzugt Methyl, Ethyl, Propyl, 1-Methyl-1-ethyl, Butyl, 1-Methyl-1-pro und 2-Methyl-1-propyl;
   - R¹⁰: Wasserstoff oder C₁-C₄-Alkyl;
   besonders bevorzugt C₁-C₄-Alkyl;
   insbesondere bevorzugt Methyl, Ethyl oder Propyl;
   - R¹¹: Wasserstoff oder C₁-C₄-Alkyl;
   besonders bevorzugt Wasserstoff oder Methyl;

Insbesondere bevorzugt sind die 3-(Heterocyclyl)-benzoylpyrazol-Derivate der Formel I, wobei
- X: O;
- R¹: Halogen oder C₁-C₄-Alkoxy; besonders bevorzugt Halogen wie Fluor, Chlor oder Brom, oder Methoxy oder Ethoxy;
insbesondere bevorzugt Chlor oder Methoxy;
- R⁶: C₁-C₄-Alkylsulfonyl; besonders bevorzugt Methylsulfonyl, Ethylsulfonyl, 1-Methyl-1-ethylsulfonyl oder Propylsulfonyl;
- R⁷: Hydroxy, C₁-C₆-Alkylsulfonyloxy, C₁-C₆-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy oder C₁-C₄-Halogenalkoxy;
besonders bevorzugt Hydroxy;
- R⁸ ,R⁹: C₁-C₄-Alkyl;
- R¹⁰: Wasserstoff oder C₁-C₄-Alkyl;
bedeuten.

Außerordentlich bevorzugt sind die 3-(Heterocyclyl)-benzoylpyrazol-Derivate der Formel I, wobei
- R⁸: C₂-C₄-Alkyl, z.B. Ethyl, 1-Methyl-1-ethyl, Propyl oder Butyl;
- R⁹: C₁-C₄-Alkyl, z.B. Methyl oder Ethyl;
- R¹⁰: Wasserstoff oder C₁-C₄-Alkyl, z.B. Methyl oder Ethyl;
bedeuten.

Ebenso außerordentlich bevorzugt sind die 3-(Heterocyclyl)-benzoylpyrazol-Derivate der Formel I, wobei
- R⁸: Methyl;
- R⁹: C₁-C₄-Alkyl, z.B. Methyl, Ethyl, Propyl oder Butyl;
- R¹⁰: C₁-C₄-Alkyl, z.B. Methyl oder Ethyl;
bedeuten.

Ebenso außerordentlich bevorzugt sind die 3-(Heterocyclyl)-benzoylpyrazolyl-Derivate der Formel I, wobei
- R⁸ ,R⁹: Methyl;
- R¹⁰: Wasserstoff;
bedeuten.
- Ebenso bevorzugt sind die 3-(Heterocyclyl)-benzoylpyrazol-Derivate der Formel I, in der die Variablen folgende Bedeutung haben:
   - X: O
   - R¹: Nitro, Halogen, C₁-C₄-Alkoxy oder C₁-C₄-Alkylthio; besonders bevorzugt Nitro, Halogen oder C₁-C₄-Alkoxy;
   insbesondere bevorzugt Halogen, wie Fluor, Chlor oder Brom, oder C₁-C₄-Alkoxy, wie Methoxy oder Ethoxy;
   insbesonderst bevorzugt Chlor oder Methoxy;
   - R² ,R³ ,R⁴ ,R⁵: Wasserstoff, C₁-C₄-Alkyl oder C₁-C₄-Halogenalkyl; besonders bevorzugt Wasserstoff, Methyl, Ethyl, Propyl, 1-Methyl-1-ethyl, Chlormethyl oder Fluormethyl;
   insbesondere bevorzugt Wasserstoff, Methyl, Ethyl oder Chlormethyl;
   - R⁶: Halogen, Nitro, C₁-C₄-Halogenalkyl, C₁-C₄-Alkoxy oder C₁-C₄-Halogenalkoxy;
   besonders bevorzugt Halogen, wie Chlor oder Brom, Nitro, C₁-C₂-Halogenalkyl, wie Difluormethyl oder Trifluormethyl, C₁-C₂-Alkoxy oder C₁-C₂-Halogenalkoxy, wie Difluormethoxy, Chlordifluormethoxy oder Trifluormethoxy;
   - R⁷: Hydroxy, C₁-C₆ -Alkoxy, C₃-C₆-Alkenyloxy, C₁-C₆-Alkylsulfonyloxy, C₁-C₆-Alkylcarbonyloxy, C₁-C₄-(Alkylthio)carbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy;
   besonders bevorzugt Hydroxy, C₁-C₄-Alkoxy, C₃-C₆-Alkenyloxy, C₁-C₄-Alkylsulfonyloxy, C₁-C₄-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy;
   - R⁸ ,R⁹: C₁-C₄-Alkyl;
   besonders bevorzugt Methyl, Ethyl, Propyl, 1-Methyl-1-eth Butyl, 1-Methyl-1-propyl und 2-Methyl-1-propyl;
   - R¹⁰: Wasserstoff oder C₁-C₄-Alkyl;
   besonders bevorzugt C₁-C₄-Alkyl;
   insbesondere bevorzugt Methyl, Ethyl oder Propyl;
   - R¹¹: Wasserstoff oder C₁-C₄-Alkyl;
   besonders bevorzugt Wasserstoff oder Methyl;

Insbesondere bevorzugt sind die 3-(Heterocyclyl)-benzoylpyrazol-Derivate der Formel I, wobei
- X: O;
- R¹: Halogen oder C₁-C₄-Alkoxy;
besonders bevorzugt Halogen wie Fluor, Chlor oder Brom, oder Methoxy oder Ethoxy;
insbesondere bevorzugt Chlor oder Methoxy;
- R⁶: Halogen, Nitro, C₁-C₄-Halogenalkyl, C₁-C₄-Alkoxy oder C₁-C₄-Halogenalkoxy;
besonders bevorzugt Halogen, wie Chlor oder Brom, Nitro, C₁-C₂-Halogenalkyl, wie Difluormethyl oder Trifluormethyl, C₁-C₂-Alkoxy oder C₁-C₂-Halogenalkoxy, wie Difluormethoxy;
- R⁷: Hydroxy, C₁-C₆-Alkylsulfonyloxy, C₁-C₆-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy oder C₁-C₄-Halogenalkoxy;
besonders bevorzugt Hydroxy;
- R⁸ ,R⁹: C₁-C₄-Alkyl;
- R¹⁰: Wasserstoff oder C₁-C₄-Alkyl;
bedeuten.

Außerordentlich bevorzugt sind die 3-(Heterocyclyl)-benzoylpyrazol-Derivate der Formel I, wobei
- R⁸: C₂-C₄-Alkyl, z.B. Ethyl, 1-Methyl-1-ethyl, Propyl oder Butyl;
- R⁹: C₁-C₄-Alkyl, z.B. Methyl oder Ethyl;
- R¹⁰: Wasserstoff oder C₁-C₄-Alkyl, z.B. Methyl oder Ethyl;
bedeuten.

Ebenso außerordentlich bevorzugt sind die 3-(Heterocyclyl)-benzoylpyrazol-Derivate der Formel I, wobei
- R⁸: Methyl;
- R⁹: C₁-C₄-Alkyl, z.B. Methyl, Ethyl, Propyl oder Butyl;
- R¹⁰: C₁-C₄-Alkyl, z.B. Methyl oder Ethyl;
bedeuten.

Ebenso außerordentlich bevorzugt sind die 3-(Heterocyclyl)-benzoylpyrazolyl-Derivate der Formel I, wobei
- R⁸ ,R⁹: Methyl;
- R¹⁰: Wasserstoff;
bedeuten.
- Ebenso bevorzugt sind die 3-(Heterocyclyl)-substituierten Benzoylpyrazole der Formel I, in der die Variablen folgende Bedeutung haben:
   - X: N(C₁-C₆-Alkyl);
   besonders bevorzugt N-Methyl, N-Ethyl, N-(1-Methyl-1-ethyl) oder N-Propyl;
   - R¹: Nitro, Halogen, C₁-C₄-Alkoxy oder C₁-C₄-Alkylthio; besonders bevorzugt Nitro, Halogen oder C₁-C₄-Alkoxy;
   insbesondere bevorzugt Halogen, wie Fluor, Chlor oder Brom, oder C₁-C₄-Alkoxy, wie Methoxy oder Ethoxy;
   insbesonderst bevorzugt Chlor oder Methoxy;
   - R² ,R³ ,R⁴ ,R⁵: Wasserstoff, C₁-C₄-Alkyl oder C₁-C₄-Halogenalkyl; besonders bevorzugt Wasserstoff, Methyl, Ethyl, Propyl, 1-Methyl-1-ethyl, Fluormethyl oder Chlormethyl;
   insbesondere bevorzugt Wasserstoff, Methyl, Ethyl oder Chlormethyl;
   - R⁶: Halogen, Nitro, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfonyl oder C₁-C₄-Halogenalkylsulfonyl;
   besonders bevorzugt Halogen, wie Fluor, Chlor oder Brom, Nitro, C₁-C₄-Halogenalkyl, wie Difluormethyl oder Trifluormethyl, C₁-C₄-Alkoxy, wie Methoxy oder Ethoxy, C₁-C₄-Halogenalkoxy wie Difluormethoxy, Chlordifluormethoxy oder Trifluormethoxy, C₁-C₄-Alkylthio wie Methylthio oder Ethylthio oder C₁-C₄-Alkylsulfonyl, wie Methylsulfonyl, Ethylsulfonyl, 1-Methyl-1-ethylsulfonyl oder Propylsulfonyl;
   - R⁷: Hydroxy, C₁-C₆-Alkoxy, C₃-C₆-Alkenyloxy, C₁-C₆-Alkylsulfonyloxy, C₁-C₆-Alkylcarbonyloxy, C₁-C₄-(Alkylthio) carbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy;
   besonders bevorzugt Hydroxy, C₁-C₄-Alkoxy, C₃-C₆-Alkenyloxy, C₁-C₄-Alkylsulfonyloxy, C₁-C₄-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy;
   - R⁸, R⁹: C₁-C₄-Alkyl; besonders bevorzugt Methyl, Ethyl, Propyl, 1-Methyl-2-ethyl, Butyl, 1-Methyl-1-propyl und 2-Methyl-1-propyl;
   - R¹⁰: Wasserstoff oder C₁-C₄-Alkyl;
   besonders bevorzugt C₁-C₄-Alkyl;
   insbesondere bevorzugt Methyl, Ethyl oder Propyl;
   - R¹¹: Wasserstoff oder C₁-C₄-Alkyl;
   besonders bevorzugt Wasserstoff oder Methyl;

Insbesondere bevorzugt sind die 3-(Heterocyclyl)-benzoylpyrazol-Derivate der Formel I, wobei
- R⁷: Hydroxy, C₁-C₆-Alkylsulfonyloxy, C₁-C₆-Alkylcarbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy oder C₁-C₄-Halogenalkoxy;
besonders bevorzugt Hydroxy;

- Ebenso bevorzugt sind die 3-(Heterocyclyl)-benzoylpyrazolDerivate der Formel I, in der die Variablen folgende Bedeutung haben:
   - X: O;
   - R¹: Halogen oder C₁-C₄-Alkoxy;
   besonders bevorzugt Fluor, Chlor, Brom, Methoxy oder Ethoxy;
   insbesondere bevorzugt Chlor oder Methoxy;
   - R² ,R³ ,R⁴ ,R⁵: Wasserstoff;
   - R⁶: C₁-C₄-Alkylsulfonyl;
   besonders bevorzugt Methylsulfonyl;
   - R⁷: Hydroxy, C₁-C₆-Alkoxy, C₁-C₄-(Alkylthio)carbonyloxy oder Phenylcarbonyloxy, wobei der Phenylrest partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy; besonders bevorzugt Hydroxy, C₁-C₆-Alkoxy oder Phenylcarbonyloxy, wobei der Phenylrest partiell oder vollständig halogeniert sein kann und/oder eine bis drei der folgenden Gruppen tragen kann:
   Nitro, Cyano, C₁-C₄-Alkyl, C₁-C₄-Halogenalkyl, C₁-C₄-Alkoxy oder C₁-C₄-Halogenalkoxy;
   - R⁸ ,R⁹: C₁-C₄-Alkyl;
   - R¹⁰: Wasserstoff oder C₁-C₄-Alkyl;
   - R¹¹: Wasserstoff.

Außerordentlich bevorzugt sind die Verbindungen der Formel Ia1 (≡I mit R¹ = Cl; R⁸,R⁹ = CH₃; R¹⁰,R¹¹=H), insbesondere die Verbindungen Ia1.1 bis la1.300 der Tabelle 1, wobei die Restedefinitionen X und R¹ bis R¹¹ nicht nur in Kombination miteinander, sondern auch jeweils für sich allein betrachtet für die erfindungsgemäßen Verbindungen eine besondere Bedeutung haben.

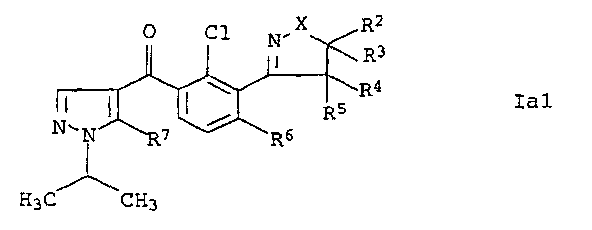

**Tabelle 1:**

| Nr. | X | R² | R³ | R⁴ | R⁵ | R⁶ | R⁷ |
|---|---|---|---|---|---|---|---|
| Ia1.1 | O | H | H | H | H | SCH₃ | OH |
| Ia1.2 | O | H | H | H | H | SCH₂CH₃ | OH |
| Ia1.3 | O | H | H | H | H | SO₂CH₃ | OH |
| Ia1.4 | O | H | H | H | H | SO₂CH₂CH₃ | OH |
| Ia1.5 | O | H | H | H | H | SO₂CH(CH₃)₂ 2 | OH |
| Ial.6 | O | H | H | H | H | SO₂(CH₂)₂CH₃ | OH |
| Ia1.7 | O | H | H | H | H | Cl | OH |
| Ia1.8 | O | H | H | H | H | Br | OH |
| Ial.9 | O | H | H | H | H | NO₂ | OH |
| Ia1.10 | O | H | H | H | H | CHF₂ | OH |
| Ia1.11 | O | H | H | H | H | CF₃ | OH |
| Ia1.12 | O | H | H | H | H | OCH₃ | OH |
| Ia1.13 | O | H | H | H | H | OCH₂CH₃ | OH |
| Ia1.14 | O | H | H | H | H | OCHF₂ | OH |
| Ia1.15 | O | H | H | H | H | OCF₃ | OH |
| Ia1.16 | O | CH₃ | H | H | H | SCH₃ | OH |
| Ia1.17 | O | CH₃ | H | H | H | SCH₂CH₃ | OH |
| Ia1.18 | O | CH₃ | H | H | H | SO₂CH₃ | OH |
| Ia1.19 | O | CH₃ | H | H | H | SO₂CH₂CH₃ | OH |
| Ia1.20 | O | CH₃ | H | H | H | SO₂CH(CH₃)₂ | OH |
| Ia1.21 | O | CH₃ | H | H | H | SO₂(CH₂)₂CH₃ | OH |
| Ia1.22 | O | CH₃ | H | H | H | Cl | OH |
| Ia1.23 | O | CH₃ | H | H | H | Br | OH |
| Ia1.24 | O | CH₃ | H | H | H | NO₂ | OH |
| Ia1.25 | O | CH₃ | H | H | H | CHF₂ | OH |
| Ia1.26 | O | CH₃ | H | H | H | CF₃ | OH |
| Ia1.27 | O | CH₃ | H | H | H | OCH₃ | OH |
| Ia1.28 | O | CH₃ | H | H | H | OCH₂CH₃ | OH |
| Ia1.29 | O | CH₃ | H | H | H | OCHF₂ | OH |
| Ia1.30 | O | CH₃ | H | H | H | OCF₃ | OH |
| Ia1.31 | O | H | H | CH₃ | H | SCH₃ | OH |
| Ia1.32 | O | H | H | CH₃ | H | SCH₂CH₃ | OH |
| Ia1.33 | O | H | H | CH₃ | H | SO₂CH₃ | OH |
| Ia1.34 | O | H | H | CH₃ | H | SO₂CH₂CH₃ | OH |
| Ia1.35 | O | H | H | CH₃ | H | SO₂CH(CH₃)₂ | OH |
| Ia1.36 | O | H | H | CH₃ | H | SO₂ (CH₂)₂ CH₃ | OH |
| Ia1.37 | O | H | H | CH₃ | H | Cl | OH |
| Ia1.38 | O | H | H | CH₃ | H | Br | OH |
| Ia1.39 | O | H | H | CH₃ | H | NO₂ | OH |
| Ia1.40 | O | H | H | CH₃ | H | CHF₂ | OH |
| Ia1.41 | O | H | H | CH₃ | H | CF₃ | OH |
| Ia1.42 | O | H | H | CH₃ | H | OCH₃ | OH |
| Ia1.43 | O | H | H | CH₃ | H | OCH₂CH₃ | OH |
| Ia1.44 | O | H | H | CH₃ | H | OCHF₂ | OH |
| Ia1.45 | O | H | H | CH₃ | H | OCF₃ | OH |
| Ia1.46 | O | CH₃ | CH₃ | H | H | SCH₃ | OH |
| Ia1.47 | O | CH₃ | CH₃ | H | H | SCH₂CH₃ | OH |
| Ia1.48 | O | CH₃ | CH₃ | H | H | SO₂CH₃ | OH |
| Ia1.49 | O | CH₃ | CH₃ | H | H | SO₂CH₂CH₃ | OH |
| Ia1.50 | O | CH₃ | CH₃ | H | H | SO₂CH(CH₃)₂ | OH |
| Ia1.51 | O | CH₃ | CH₃ | H | H | SO₂(CH₂)₂CH₃ | OH |
| Ia1.52 | O | CH₃ | CH₃ | H | H | Cl | OH |
| Ia1.53 | O | CH₃ | CH₃ | H | H | Br | OH |
| Ia1.54 | O | CH₃ | CH₃ | H | H | NO₂ | OH |
| Ia1.55 | O | CH₃ | CH₃ | H | H | CHF₂ | OH |
| Ia1.56 | O | CH₃ | CH₃ | H | H | CF₃ | OH |
| Ia1.57 | O | CH₃ | CH₃ | H | H | OCH₃ | OH |
| Ia1.58 | O | CH₃ | CH₃ | H | H | OCH₂CH₃ | OH |
| Ia1.59 | O | CH₃ | CH₃ | H | H | OCHF₂ | OH |
| Ia1.60 | O | CH₃ | CH₃ | H | H | OCF₃ | OH |
| Ia1.61 | O | CH₃ | H | CH₃ | H | SCH₃ | OH |
| Ia1.62 | O | CH₃ | H | CH₃ | H | SCH₂CH₃ | OH |
| Ia1.63 | O | CH₃ | H | CH₃ | H | SO₂CH₃ | OH |
| Ia1.64 | O | CH₃ | H | CH₃ | H | SO₂CH₂CH₃ | OH |
| Ia1.65 | O | CH₃ | H | CH₃ | H | SO₂(CH₃)₂ | OH |
| Ia1.66 | O | CH₃ | H | CH₃ | H | SO₂(CH₂)₂CH₃ | OH |
| Ia1.67 | O | CH₃ | H | CH₃ | H | Cl | OH |
| Ia1.68 | O | CH₃ | H | CH₃ | H | Br | OH |
| Ia1.69 | O | CH₃ | H | CH₃ | H | NO₂ | OH |
| Ia1.70 | O | CH₃ | H | CH₃ | H | CHF₂ | OH |
| Ia1.71 | O | CH₃ | H | CH₃ | H | CF₃ | OH |
| Ia1.72 | O | CH₃ | H | CH₃ | H | OCH₃ | OH |
| Ia1.73 | O | CH₃ | H | CH₃ | H | OCH₂CH₃ | OH |
| Ia1.74 | O | CH₃ | H | CH₃ | H | OCHF₂ | OH |
| Ia1.75 | O | CH₃ | H | CH₃ | H | OCF₃ | OH |
| Ia1.76 | O | H | H | CH₃ | CH₃ | SCH₃ | OH |
| Ia1.77 | O | H | H | CH₃ | CH₃ | SCH₂CH₃ | OH |
| Ia1.78 | O | H | H | CH₃ | CH₃ | SO₂CH₃ | OH |
| Ia1.79 | O | H | H | CH₃ | CH₃ | SO₂CH₂CH₃ | OH |
| Ia1.80 | O | H | H | CH₃ | CH₃ | SO₂CH(CH₃)₂ | OH |
| Ia1.81 | O | H | H | CH₃ | CH₃ | SO₂(CH₂)₂CH₃ | OH |
| Ia1.82 | O | H | H | CH₃ | CH₃ | Cl | OH |
| Ia1.83 | O | H | H | CH₃ | CH₃ | Br | OH |
| Ia1.84 | O | H | H | CH₃ | CH₃ | NO₂ | OH |
| Ia1.85 | O | H | H | CH₃ | CH₃ | CHF₂ | OH |
| Ia1.86 | O | H | H | CH₃ | CH₃ | CF₃ | OH |
| Ia1.87 | O | H | H | CH₃ | CH₃ | OCH₃ | OH |
| Ia1.88 | O | H | H | CH₃ | CH₃ | OCH₂CH₃ | OH |
| Ia1.89 | O | H | H | CH₃ | CH₃ | OCHF₂ | OH |
| Ia1.90 | O | H | H | CH₃ | CH₃ | OCF₃ | OH |
| Ia1.91 | O | CH₃ | CH₃ | CH₃ | H | SCH₃ | OH |
| Ia1.92 | O | CH₃ | CH₃ | CH₃ | H | SCH₂CH₃ | OH |
| Ia1.93 | O | CH₃ | CH₃ | CH₃ | H | SO₂CH₃ | OH |
| Ia1.94 | O | CH₃ | CH₃ | CH₃ | H | SO₂CH₂CH₃ | OH |
| Ia1.95 | O | CH₃ | CH₃ | CH₃ | H | SO₂CH(CH₃)₂ | OH |
| Ia1.96 | O | CH₃ | CH₃ | CH₃ | H | SO₂(CH₂)₂CH₃ | OH |
| Ia1.97 | O | CH₃ | CH₃ | CH₃ | H | Cl | OH |
| Ia1.98 | O | CH₃ | CH₃ | CH₃ | H | Br | OH |
| Ia1.99 | O | CH₃ | CH₃ | CH₃ | H | NO₂ | OH |
| Ia1.100 | O | CH₃ | CH₃ | CH₃ | H | CHF₂ | OH |
| Ia1.101 | O | CH₃ | CH₃ | CH₃ | H | CF₃ | OH |
| Ia1.102 | O | CH₃ | CH₃ | CH₃ | H | OCH₃ | OH |
| Ia1.103 | O | CH₃ | CH₃ | CH₃ | H | OCH₂CH₃ | OH |
| Ia1.104 | O | CH₃ | CH₃ | CH₃ | H | OCHF₂ | OH |
| Ia1.105 | O | CH₃ | CH₃ | CH₃ | H | OCF₃ | OH |
| Ia1.106 | O | CH₃ | H | CH₃ | CH₃ | SCH₃ | OH |
| Ia1.107 | O | CH₃ | H | CH₃ | CH₃ | SCH₂CH₃ | OH |
| Ia1.108 | O | CH₃ | H | CH₃ | CH₃ | SO₂CH₃ | OH |
| Ia1.109 | O | CH₃ | H | CH₃ | CH₃ | SO₂CH₂CH₃ | OH |
| Ia1.110 | O | CH₃ | H | CH₃ | CH₃ | SO₂CH(CH₃)₂ | OH |
| Ia1.111 | O | CH₃ | H | CH₃ | CH₃ | SO₂(CH₂)₂CH₃ | OH |
| Ia1.112 | O | CH₃ | H | CH₃ | CH₃ | Cl | OH |
| Ia1.113 | O | CH₃ | H | CH₃ | CH₃ | Br | OH |
| Ia1.114 | O | CH₃ | H | CH₃ | CH₃ | NO₂ | OH |
| Ia1.115 | O | CH₃ | H | CH₃ | CH₃ | CHF₂ | OH |
| Ia1.116 | O | CH₃ | H | CH₃ | CH₃ | CF₃ | OH |
| Ia1.117 | O | CH₃ | H | CH₃ | CH₃ | OCH₃ | OH |
| Ia1.118 | O | CH₃ | H | CH₃ | CH₃ | OCH₂CH₃ | OH |
| Ia1.119 | O | CH₃ | H | CH₃ | CH₃ | OCHF₂ | OH |
| Ia1.120 | O | CH₃ | H | CH₃ | CH₃ | OCF₃ | OH |
| Ia1.121 | O | CH₃ | CH₃ | CH₃ | CH₃ | SCH₃ | OH |
| Ia1.122 | O | CH₃ | CH₃ | CH₃ | CH₃ | SCH₂CH₃ | OH |
| Ia1.123 | O | CH₃ | CH₃ | CH₃ | CH₃ | SO₂CH₃ | OH |
| Ia1.124 | O | CH₃ | CH₃ | CH₃ | CH₃ | SO₂CH₂CH₃ | OH |
| Ia1.125 | O | CH₃ | CH₃ | CH₃ | CH₃ | SO₂CH(CH₃)₂ | OH |
| Ia1.126 | O | CH₃ | CH₃ | CH₃ | CH₃ | SO₂(CH₂)₂CH₃ | OH |
| Ia1.127 | O | CH₃ | CH₃ | CH₃ | CH₃ | Cl | OH |
| Ia1.128 | O | CH₃ | CH₃ | CH₃ | CH₃ | Br | OH |
| Ia1.129 | O | CH₃ | CH₃ | CH₃ | CH₃ | NO₂ | OH |
| Ia1.130 | O | CH₃ | CH₃ | CH₃ | CH₃ | CHF₂ | OH |
| Ia1.131 | O | CH₃ | CH₃ | CH₃ | CH₃ | CF₃ | OH |
| Ia1.132 | O | CH₃ | CH₃ | CH₃ | CH₃ | OCH₃ | OH |
| Ia1.133 | O | CH₃ | CH₃ | CH₃ | CH₃ | OCH₂CH₃ | OH |
| Ia1.134 | O | CH₃ | CH₃ | CH₃ | CH₃ | OCHF₂ | OH |
| Ia1.135 | O | CH₃ | CH₃ | CH₃ | CH₃ | OCF₃ | OH |
| Ia1.136 | O | CH₂Cl | H | H | H | SCH₃ | OH |
| Ia1.137 | O | CH₂Cl | H | H | H | SCH₂CH₃ | OH |
| Ia1.138 | O | CH₂Cl | H | H | H | SO₂CH₃ | OH |
| Ia1.139 | O | CH₂Cl | H | H | H | SO₂CH₂CH₃ | OH |
| Ia1.140 | O | CH₂Cl | H | H | H | SO₂CH(CH₃)₂ | OH |
| Ia1.141 | O | CH₂Cl | H | H | H | SO₂(CH₂)₂CH₃ | OH |
| Ia1.142 | O | CH₂Cl | H | H | H | Cl | OH |
| Ia1.143 | O | CH₂Cl | H | H | H | Br | OH |
| Ia1.144 | O | CH₂Cl | H | H | H | NO₂ | OH |
| Ia1.145 | O | CH₂Cl | H | H | H | CHF₂ | OH |
| Ia1.146 | O | CH₂Cl | H | H | H | CF₃ | OH |
| Ia1.147 | O | CH₂Cl | H | H | H | OCH₃ | OH |
| Ia1.148 | O | CH₂Cl | H | H | H | OCH₂CH₃ | OH |
| Ia1.149 | O | CH₂Cl | H | H | H | OCHF₂ | OH |
| Ia1.150 | O | CH₂Cl | H | H | H | OCF₃ | OH |
| Ia1.151 | NCH₃ | H | H | H | H | SCH₃ | OH |
| Ia1.152 | NCH₃ | H | H | H | H | SCH₂CH₃ | OH |
| Ia1.153 | NCH₃ | H | H | H | H | SO₂CH₃ | OH |
| Ia1.154 | NCH₃ | H | H | H | H | SO₂CH₂CH₃ | OH |
| Ia1.155 | NCH₃ | H | H | H | H | SO₂CH(CH₃)₂ | OH |
| Ia1.156 | NCH₃ | H | H | H | H | SO₂(CH₂)₂CH₃ | OH |
| Ia1.157 | NCH₃ | H | H | H | H | Cl | OH |
| Ia1.158 | NCH₃ | H | H | H | H | Br | OH |
| Ia1.159 | NCH₃ | H | H | H | H | NO₂ | OH |
| Ia1.160 | NCH₃ | H | H | H | H | CHF₂ | OH |
| Ia1.161 | NCH₃ | H | H | H | H | CF₃ | OH |
| Ia1.162 | NCH₃ | H | H | H | H | OCH₃ | OH |
| Ia1.163 | NCH₃ | H | H | H | H | OCH₂CH₃ | OH |
| Ia1.164 | NCH₃ | H | H | H | H | OCHF₂ | OH |
| Ia1.165 | NCH₃ | H | H | H | H | OCF₃ | OH |
| Ia1.166 | NCH₃ | CH₃ | H | H | H | SCH₃ | OH |
| Ia1.167 | NCH₃ | CH₃ | H | H | H | SCH₂CH₃ | OH |
| Ia1.168 | NCH₃ | CH₃ | H | H | H | SO₂CH₃ | OH |
| Ia1.169 | NCH₃ | CH₃ | H | H | H | SO₂CH₂CH₃ | OH |
| Ia1.170 | NCH₃ | CH₃ | H | H | H | SO₂CH(CH₃)₂ | OH |
| Ia1.171 | NCH₃ | CH₃ | H | H | H | SO₂(CH₂)₂CH₃ | OH |
| Ia1.172 | NCH₃ | CH₃ | H | H | H | Cl | OH |
| Ia1.173 | NCH₃ | CH₃ | H | H | H | Br | OH |
| Ia1.174 | NCH3 | CH3 | H | H | H | NO₂ | OH |
| Ia1.175 | NCH₃ | CH₃ | H | H | H | CHF₂ | OH |
| Ia1.176 | NCH₃ | CH₃ | H | H | H | CF₃ | OH |
| Ia1.177 | NCH₃ | CH₃ | H | H | H | OCH₃ | OH |
| Ia1.178 | NCH₃ | CH₃ | H | H | H | OCH₂CH₃ | OH |
| Ia1.179 | NCH₃ | CH₃ | H | H | H | OCHF₂ | OH |
| Ia1.180 | NCH₃ | CH₃ | H | H | H | OCF₃ | OH |
| Ia1.181 | NCH₃ | H | H | CH₃ | H | SCH₃ | OH |
| Ia1.182 | NCH₃ | H | H | CH₃ | H | SCH₂CH₃ | OH |
| Ia1.183 | NCH₃ | H | H | CH₃ | H | SO₂CH₃ | OH |
| Ia1.184 | NCH₃ | H | H | CH₃ | H | SO₂CH₂CH₃ | OH |
| Ia1.185 | NCH₃ | H | H | CH₃ | H | SO₂CH(CH₃)₂ | OH |
| Ia1.186 | NCH₃ | H | H | CH₃ | H | SO₂(CH₂)₂CH₃ | OH |
| Ia1.187 | NCH₃ | H | H | CH₃ | H | Cl | OH |
| Ia1.188 | NCH₃ | H | H | CH₃ | H | Br | OH |
| Ia1.189 | NCH₃ | H | H | CH₃ | H | NO₂ | OH |
| Ia1.190 | NCH₃ | H | H | CH₃ | H | CHF₂ | OH |
| Ia1.191 | NCH₃ | H | H | CH₃ | H | CF₃ | OH |
| Ia1.192 | NCH₃ | H | H | CH₃ | H | OCH₃ | OH |
| Ia1.193 | NCH₃ | H | H | CH₃ | H | OCH₂CH₃ | OH |
| Ia1.194 | NCH₃ | H | H | CH₃ | H | OCHF₂ | OH |
| Ia1.195 | NCH₃ | H | H | CH₃ | H | OCF₃ | OH |
| Ia1.196 | NCH₃ | CH₃ | CH₃ | H | H | SCH₃ | OH |
| Ia1.197 | NCH₃ | CH₃ | CH₃ | H | H | SCH₂CH₃ | OH |
| Ia1.198 | NCH₃ | CH₃ | CH₃ | H | H | SO₂CH₃ | OH |
| Ia1.199 | NCH₃ | CH₃ | CH₃ | H | H | SO₂CH₂CH₃ | OH |
| Ia1.200 | NCH₃ | CH₃ | CH₃ | H | H | SO₂CH(CH₃)₂ | OH |
| Ia1.201 | NCH₃ | CH₃ | CH₃ | H | H | SO₂(CH₂)₂CH₃ | OH |
| Ia1.202 | NCH₃ | CH₃ | CH₃ | H | H | Cl | OH |
| Ia1.203 | NCH₃ | CH₃ | CH₃ | H | H | Br | OH |
| Ia1.204 | NCH₃ | CH₃ | CH₃ | H | H | NO₂ | OH |
| Ia1.205 | NCH₃ | CH₃ | CH₃ | H | H | CHF₂ | OH |
| Ia1.206 | NCH₃ | CH₃ | CH₃ | H | H | CF₃ | OH |
| Ia1.207 | NCH₃ | CH₃ | CH₃ | H | H | OCH₃ | OH |
| Ia1.208 | NCH₃ | CH₃ | CH₃ | H | H | OCH₂CH₃ | OH |
| Ia1.209 | NCH₃ | CH₃ | CH₃ | H | H | OCHF₂ | OH |
| Ia1.210 | NCH₃ | CH₃ | CH₃ | H | H | OCF₃ | OH |
| Ia1.211 | NCH₃ | CH₃ | H | CH₃ | H | SCH₃ | OH |
| Ia1.212 | NCH₃ | CH₃ | H | CH₃ | H | SCH₂CH₃ | OH |
| Ia1.213 | NCH₃ | CH₃ | H | CH₃ | H | SO₂CH₃ | OH |
| Ia1.214 | NCH₃ | CH₃ | H | CH₃ | H | SO₂CH₂CH₃ | OH |
| Ia1.215 | NCH₃ | CH₃ | H | CH₃ | H | SO₂CH(CH₃)₂ | OH |
| Ia1.216 | NCH₃ | CH₃ | H | CH₃ | H | SO₂(CH₂)₂CH₃ | OH |
| Ia1.217 | NCH₃ | CH₃ | H | CH₃ | H | Cl | OH |
| Ia1.218 | NCH₃ | CH₃ | H | CH₃ | H | Br | OH |
| Ia1.219 | NCH₃ | CH₃ | H | CH₃ | H | NO₂ | OH |
| Ia1.220 | NCH₃ | CH₃ | H | CH₃ | H | CHF₂ | OH |
| Ia1.221 | NCH₃ | CH₃ | H | CH₃ | H | CF₃ | OH |
| Ia1.222 | NCH₃ | CH₃ | H | CH₃ | H | OCH₃ | OH |
| Ia1.223 | NCH₃ | CH₃ | H | CH₃ | H | OCH₂CH₃ | OH |
| Ia1.224 | NCH₃ | CH₃ | H | CH₃ | H | OCHF₂ | OH |
| Ia1.225 | NCH₃ | CH₃ | H | CH₃ | H | OCF₃ | OH |
| Ia1.226 | NCH₃ | H | H | CH₃ | CH₃ | SCH₃ | OH |
| Ia1.227 | NCH₃ | H | H | CH₃ | CH₃ | SCH₂CH₃ | OH |
| Ia1.228 | NCH₃ | H | H | CH₃ | CH₃ | SO₂CH₃ | OH |
| Ia1.229 | NCH₃ | H | H | CH₃ | CH₃ | SO₂CH₂CH₃ | OH |
| Ia1.230 | NCH₃ | H | H | CH₃ | CH₃ | SO₂CH(CH₃)₂ | OH |
| Ia1.231 | NCH₃ | H | H | CH₃ | CH₃ | SO₂(CH₂)₂CH₃ | OH |
| Ia1.232 | NCH₃ | H | H | CH₃ | CH₃ | Cl | OH |
| Ia1.233 | NCH₃ | H | H | CH₃ | CH₃ | Br | OH |
| Ia1.234 | NCH₃ | H | H | CH₃ | CH₃ | NO₂ | OH |
| Ia1.235 | NCH₃ | H | H | CH₃ | CH₃ | CHF₂ | OH |
| Ia1.236 | NCH₃ | H | H | CH₃ | CH₃ | CF₃ | OH |
| Ia1.237 | NCH₃ | H | H | CH₃ | CH₃ | OCH₃ | OH |
| Ia1.238 | NCH₃ | H | H | CH₃ | CH₃ | OCH₂CH₃ | OH |
| Ia1.239 | NCH₃ | H | H | CH₃ | CH₃ | OCHF₂ | OH |
| Ia1.240 | NCH₃ | H | H | CH₃ | CH₃ | OCF₃ | OH |
| Ia1.241 | NCH₃ | CH₃ | CH₃ | CH₃ | H | SCH₃ | OH |
| Ia1.242 | NCH₃ | CH₃ | CH₃ | CH₃ | H | SCH₂CH₃ | OH |
| Ia1.243 | NCH₃ | CH₃ | CH₃ | CH₃ | H | SO₂CH₃ | OH |
| Ia1.244 | NCH₃ | CH₃ | CH₃ | CH₃ | H | SO₂CH₂CH₃ | OH |
| Ia1.245 | NCH₃ | CH₃ | CH₃ | CH₃ | H | SO₂CH(CH₃)₂ | OH |
| Ia1.246 | NCH₃ | CH₃ | CH₃ | CH₃ | H | SO₂(CH₂)₂CH₃ | OH |
| Ia1.247 | NCH₃ | CH₃ | CH₃ | CH₃ | H | Cl | OH |
| Ia1.248 | NCH₃ | CH₃ | CH₃ | CH₃ | H | Br | OH |
| Ia1.249 | NCH₃ | CH₃ | CH₃ | CH₃ | H | NO₂ | OH |
| Ia1.250 | NCH₃ | CH₃ | CH₃ | CH₃ | H | CHF₂ | OH |
| Ia1.251 | NCH₃ | CH₃ | CH₃ | CH₃ | H | CF₃ | OH |
| Ia1.252 | NCH₃ | CH₃ | CH₃ | CH₃ | H | OCH₃ | OH |
| Ia1.253 | NCH₃ | CH₃ | CH₃ | CH₃ | H | OCH₂CH₃ | OH |
| Ia1.254 | NCH₃ | CH₃ | CH₃ | CH₃ | H | OCHF₂ | OH |
| Ia1.255 | NCH₃ | CH₃ | CH₃ | CH₃ | H | OCF₃ | OH |
| Ia1.256 | NCH₃ | CH₃ | H | CH₃ | CH₃ | SCH₃ | OH |
| Ia1.257 | NCH₃ | CH₃ | H | CH₃ | CH₃ | SCH₂CH₃ | OH |
| Ia1.258 | NCH₃ | CH₃ | H | CH₃ | CH₃ | SO₂CH₃ | OH |
| Ia1.259 | NCH₃ | CH₃ | H | CH₃ | CH₃ | SO₂CH₂CH₃ | OH |
| Ia1.260 | NCH₃ | CH₃ | H | CH₃ | CH₃ | SO₂CH(CH₃)₂ | OH |
| Ia1.261 | NCH₃ | CH₃ | H | CH₃ | CH₃ | SO₂(CH₂)₂CH₃ | OH |
| Ia1.262 | NCH₃ | CH₃ | H | CH₃ | CH₃ | Cl | OH |
| Ia1.263 | NCH₃ | CH₃ | H | CH₃ | CH₃ | Br | OH |
| Ia1.264 | NCH₃ | CH₃ | H | CH₃ | CH₃ | NO₂ | OH |
| Ia1.265 | NCH₃ | CH₃ | H | CH₃ | CH₃ | CHF₂ | OH |
| Ia1.266 | NCH₃ | CH₃ | H | CH₃ | CH₃ | CF₃ | OH |
| Ia1.267 | NCH₃ | CH₃ | H | CH₃ | CH₃ | OCH₃ | OH |
| Ia1.268 | NCH₃ | CH₃ | H | CH₃ | CH₃ | OCH₂CH₃ | OH |
| Ia1.269 | NCH₃ | CH₃ | H | CH₃ | CH₃ | OCHF₂ | OH |
| Ia1.270 | NCH₃ | CH₃ | H | CH₃ | CH₃ | OCF₃ | OH |
| Ia1.271 | NCH₃ | CH₃ | CH₃ | CH₃ | CH₃ | SCH₃ | OH |
| Ia1.272 | NCH₃ | CH₃ | CH₃ | CH₃ | CH₃ | SCH₂CH₃ | OH |
| Ia1.273 | NCH₃ | CH₃ | CH₃ | CH₃ | CH₃ | SO₂CH₃ | OH |
| Ia1.274 | NCH₃ | CH₃ | CH₃ | CH₃ | CH₃ | SO₂CH₂CH₃ | OH |
| Ia1.275 | NCH₃ | CH₃ | CH₃ | CH₃ | CH₃ | SO₂CH(CH₂)₂ | OH |
| Ia1.276 | NCH₃ | CH₃ | CH₃ | CH₃ | CH₃ | SO₂(CH₂)₂CH₃ | OH |
| Ia1.277 | NCH₃ | CH₃ | CH₃ | CH₃ | CH₃ | Cl | OH |
| Ia1.278 | NCH₃ | CH₃ | CH₃ | CH₃ | CH₃ | Br | OH |
| Ia1.279 | NCH₃ | CH₃ | CH₃ | CH₃ | CH₃ | NO₂ | OH |
| Ia1.280 | NCH₃ | CH₃ | CH₃ | CH₃ | CH₃ | CHF₂ | OH |
| Ia1.281 | NCH₃ | CH₃ | CH₃ | CH₃ | CH₃ | CF₃ | OH |
| Ia1.282 | NCH₃ | CH₃ | CH₃ | CH₃ | CH₃ | OCH₃ | OH |
| Ia1.283 | NCH₃ | CH₃ | CH₃ | CH₃ | CH₃ | OCH₂CH₃ | OH |
| Ia1.284 | NCH₃ | CH₃ | CH₃ | CH₃ | CH₃ | OCHF₂ | OH |
| Ia1.285 | NCH₃ | CH₃ | CH₃ | CH₃ | CH₃ | OCF₃ | OH |
| Ia1.286 | NCH₃ | CH₂Cl | H | H | H | SCH₃ | OH |
| Ia1.287 | NCH₃ | CH₂Cl | H | H | H | SCH₂CH₃ | OH |
| Ia1.288 | NCH₃ | CH₂Cl | H | H | H | SO₂CH₃ | OH |
| Ia1.289 | NCH₃ | CH₂Cl | H | H | H | SC₂CH₂CH₃ | OH |
| Ia1.290 | NCH₃ | CH₂Cl | H | H | H | SO₂CH(CH₃)₂ | OH |
| Ia1.291 | NCH₃ | CH₂Cl | H | H | H | SO₂(CH₂)₂CH₃ | OH |
| Ia1.292 | NCH₃ | CH₂Cl | H | H | H | Cl | OH |
| Ia1.293 | NCH₃ | CH₂Cl | H | H | H | Br | OH |
| Ia1.294 | NCH₃ | CH₂Cl | H | H | H | NO₂ | OH |
| Ia1.295 | NCH₃ | CH₂Cl | H | H | H | CHF₂ | OH |
| Ia1.296 | NCH₃ | CH₂Cl | H | H | H | CF₃ | OH |
| Ia1.297 | NCH₃ | CH₂Cl | H | H | H | OCH₃ | OH |
| Ia1.298 | NCH₃ | CH₂Cl | H | H | H | OCH₂CH₃ | OH |
| Ia1.299 | NCH₃ | CH₂Cl | H | H | H | OCHF₂ | OH |
| Ia1.300 | NCH₃ | CH₂Cl | H | H | H | OCF₃ | OH |

Ebenso außerordentlichst bevorzugt sind die Verbindungen der Forme1 Ia2, insbesondere die Verbindungen Ia2.1 bis Ia2.300, die sich von den entsprechenden Verbindungen Ia1.1 bis Ia1.300 dadurch unterscheiden, daß R¹¹ für Methyl steht.

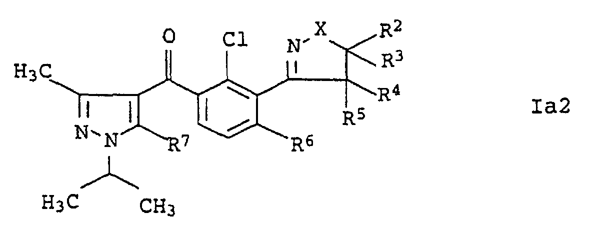

Ebenso außerordentlichst bevorzugt sind die Verbindungen der Formel Ia3, insbesondere die Verbindungen Ia3.1 bis Ia3.300, die sich von den Verbindungen Ia1.1 bis Ia1.3 dadurch unterscheiden, daß R⁸ für Ethyl steht.

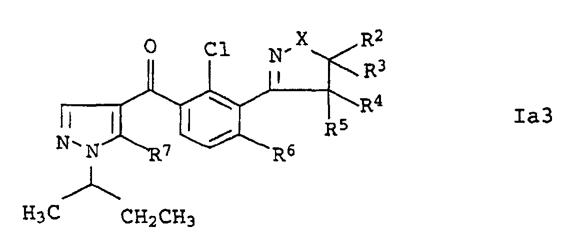

Ebenso außerordentlichst bevorzugt sind die Verbindungen der Formel Ia4, insbesondere die Verbindungen Ia4.1 bis Ia4.300, die sich von den Verbindungen Ia1.1 bis Ia1.300 dadurch unterscheiden, daß R⁸ für Ethyl und R¹¹ für Methyl stehen.

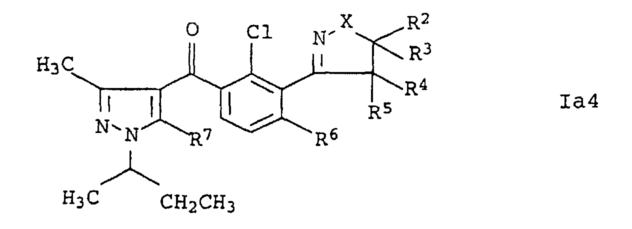

Ebenso außerordentlichst bevorzugt sind die Verbindungen der Formel Ia5, insbesondere die Verbindungen Ia5.1 bis Ia5.300, die sich von den Verbindungen Ia1.1 bis Ia1.300 dadurch unterscheiden, daß R⁸ für 1-Methyl-1-ethyl steht.

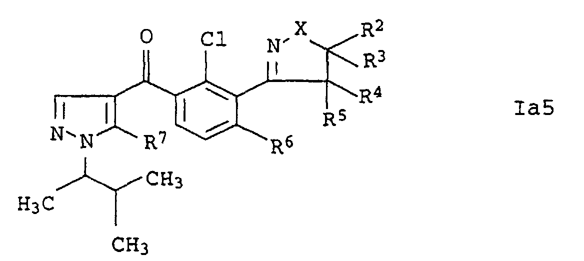

Ebenso außerordentlichst bevorzugt sind die Verbindungen der Formel Ia6, insbesondere die Verbindungen Ia6.1 bis Ia6.300, die sich von den Verbindungen Ia1.1 bis Ia1.300 dadurch unterscheiden, daß R⁸ für 1-Methyl-1-ethyl und R¹¹ für Methyl stehen.

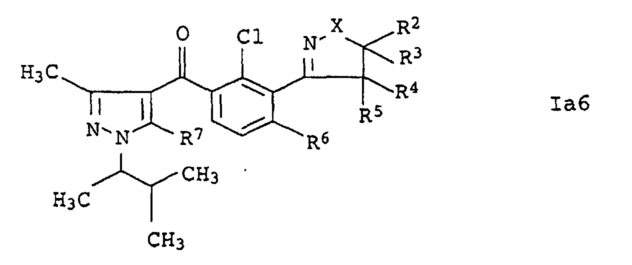

Ebenso außerordentlichst bevorzugt sind die Verbindungen der Formel Ia7, insbesondere die Verbindungen Ia7.1 bis Ia7.300, die sich von den Verbindungen Ia1.1 bis Ia1.300 davon unterscheiden, daß R¹⁰ für Methyl steht.

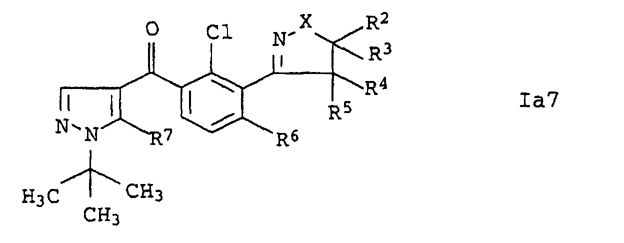

Ebenso außerordentlichst bevorzugt sind die Verbindungen der Formel Ia8, insbesondere die Verbindungen Ia8.1 bis Ia8.300, die sich von den Verbindungen Ia1.1 bis Ia1.300 dadurch unterscheiden, daß R¹⁰ für Methyl und R¹¹ für Methyl stehen.

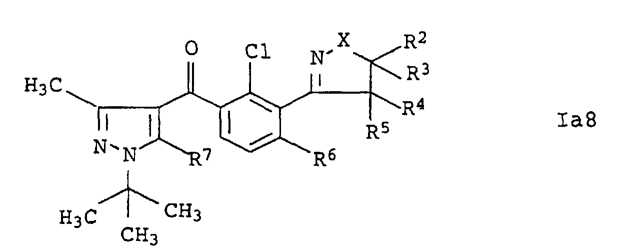

Ebenso außerordentlichst bevorzugt sind die Verbindungen der Formel Ia9, insbesondere die Verbindungen Ia9.1 bis Ia9.300, die sich von den Verbindungen Ia1.1 bis Ia1.300 dadurch unterscheiden, daß R⁸ und R⁹ für 1-Methyl-1-ethyl stehen.

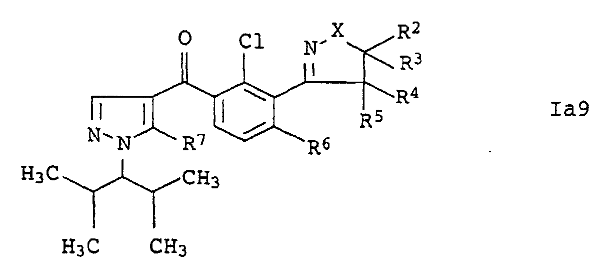

Ebenso außerordentlichst bevorzugt sind die Verbindungen der Formel Ia10, insbesondere die Verbindungen Ia10.1 bis Ia10.300, die sich von den Verbindungen Ia1.1 bis Ia1.300 dadurch unterscheiden, daß R⁸ und R⁹ für 1-Methyl-1-ethyl und R¹¹ für-Methyl stehen.

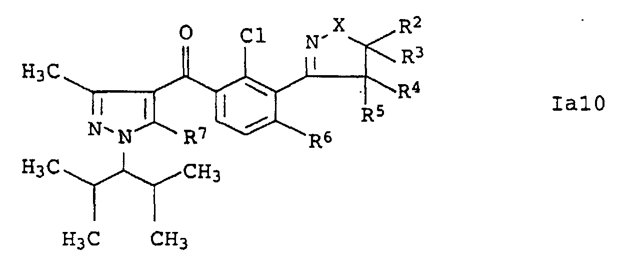

Ebenso außerordentlichst bevorzugt sind die Verbindungen der Formel Ia11, insbesondere die Verbindungen Ia11.1 bis Ia11.300, die sich von den Verbindungen Ia1.1 bis Ia1.300 dadurch unterscheiden, daß R⁸ für Ethyl und R¹⁰ für Methyl stehen.

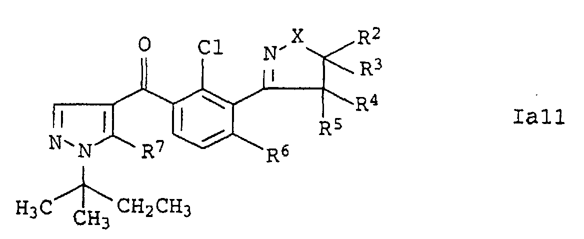

Ebenso außerordentlichst bevorzugt sind die Verbindungen der Formel 1a12, insbesondere die Verbindungen Ia12.1 bis Ia12.300, die sich von den Verbindungen Ia1.1 bis Ia1.300 dadurch unterscheiden, daß R⁸ für Ethyl und R¹⁰ und R¹¹ für Methyl stehen.

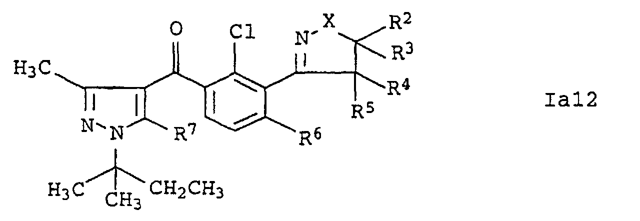

Ebenso außerordentlichst bevorzugt sind die Verbindungen der Formel Ia13, insbesondere die Verbindungen Ia13.1 bis Ia13.30 die sich von den entsprechenden Verbindungen Ia1.1 bis Ia1.300 dadurch unterscheiden, daß R¹ für Methoxy steht.

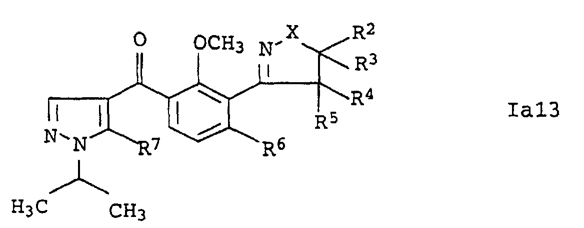

Ebenso außerordentlichst bevorzugt sind die Verbindungen der Formel Ia14, insbesondere die Verbindungen Ia14.1 bis Ia14.300, die sich von den entsprechenden Verbindungen Ia1.1 bis Ia1.300 dadurch unterscheiden, daß R¹ für Methoxy und R¹¹ für Methyl stehen.

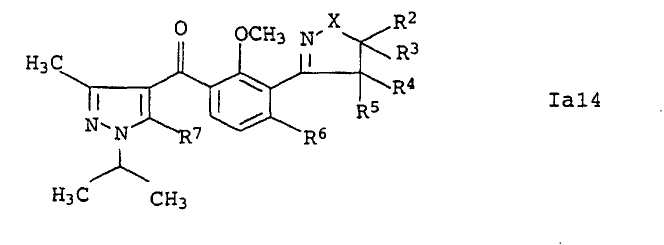

Ebenso außerordentlichst bevorzugt sind die Verbindungen der Formel Ia15, insbesondere die Verbindungen Ia15.1 bis Ia15.300, die sich von den Verbindungen Ia1.1 bis Ia1.300 dadurch unterscheiden, daß R¹ für Methoxy und R⁸ für Ethyl stehen.

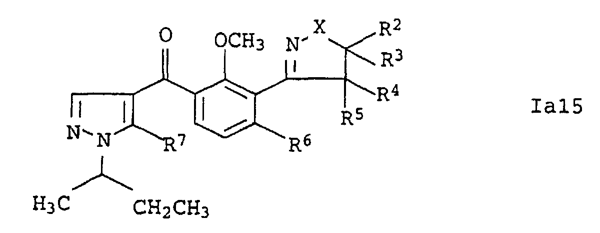

Ebenso außerordentlichst bevorzugt sind die Verbindungen der Formel Ia1 insbesondere die Verbindungen Ial6.1 bis Ia16.30 die sich von den Verbindungen Ia1.1 bis Ia1.300 dadurch unterscheiden, daß R¹ für Methoxy, R⁸ für Ethyl und R¹¹ für Methyl stehen.

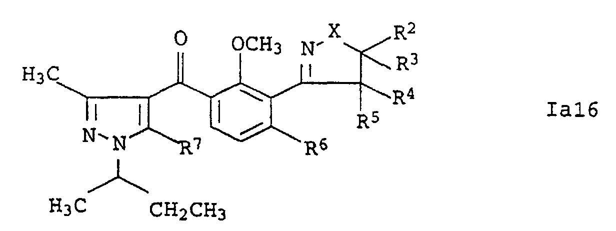

Ebenso außerordentlichst bevorzugt sind die Verbindungen der Formel Ia17, insbesondere die Verbindungen Ia17.1 bis Ia17.300, die sich von den Verbindungen Ia1.1 bis Ia1.300 dadurch unterscheiden, daß R¹ für Methoxy und R⁸ für 1-Methyl-1-ethyl stehen.

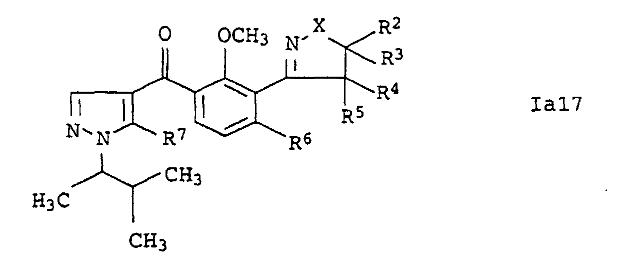

Ebenso außerordentlichst bevorzugt sind die Verbindungen der Formel Ia18, insbesondere die Verbindungen Ia18.1 bis Ia18.300, die sich von den Verbindungen Ia1.1 bis Ial.300 dadurch unterscheiden, daß R¹ für Methoxy R⁸ für 1-Methyl-1-ethyl und R¹¹ für Methyl stehen.

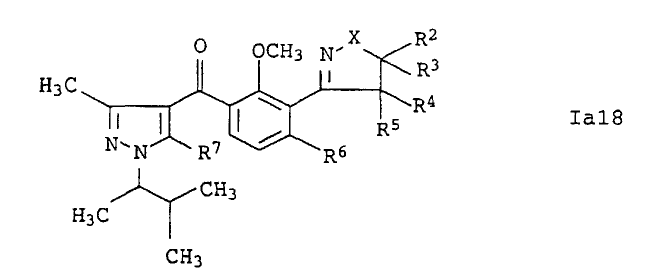

Ebenso außerordentlichst bevorzugt sind die Verbindungen der Formel Ial9, insbesondere die Verbindungen Ia19.1 bis Ia19.300, die sich von den Verbindungen Ia1.1 bis Ial.300 dadurch unterscheiden, daß R¹ für Methoxy und R¹⁰ für Methyl stehen.

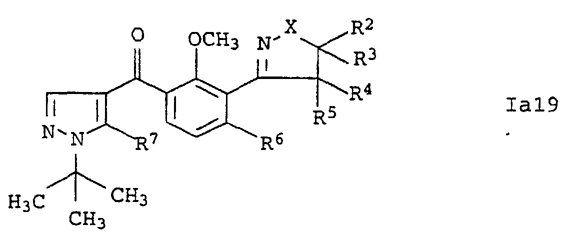

Ebenso außerordentlichst bevorzugt sind die Verbindungen der Formel Ia20, insbesondere die Verbindungen Ia20.1 bis Ia20.300, die sich von den Verbindungen Ia1.1 bis Ia1.300 dadurch unterscheiden, daß R¹ für Methoxy, R¹⁰ für Methyl und R¹¹ für Methyl stehen.

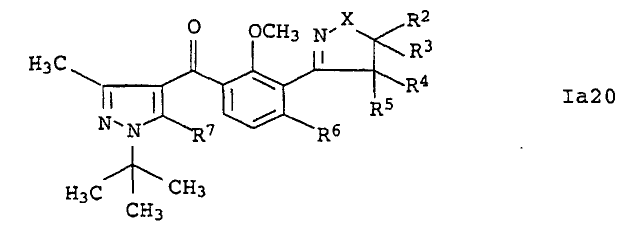

Ebenso außerordentlichst bevorzugt sind die Verbindungen der Formel Ia21, insbesondere die Verbindungen Ia21.1 bis Ia21.300, die sich von den Verbindungen Ia1.1 bis Ia1.300 dadurch unterscheiden, daß R¹ für Methoxy und R⁸ und R⁹ für 1-Methyl-1-ethyl stehen.

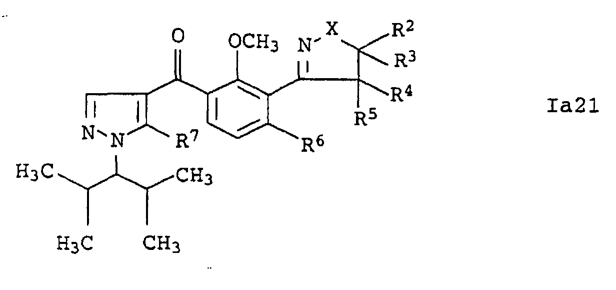

Ebenso außerordentlichst bevorzugt sind die Verbindungen der Formel Ia22, insbesondere die Verbindungen Ia22.1 bis Ia22.300, die sich von den Verbindungen Ia1.1 bis Ia1.300 dadurch unterscheiden, daß R¹ für Methoxy, R⁸ und R⁹ für 1-Methyl-1-ethyl und R¹¹ für Methyl stehen.

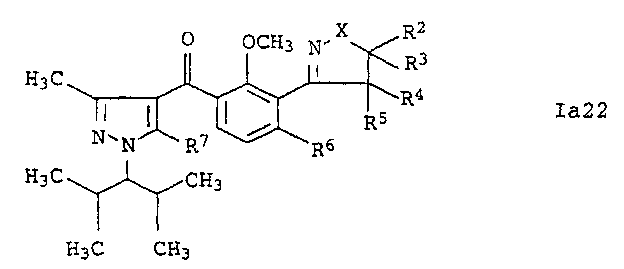

Ebenso außerordentlichst bevorzugt sind die Verbindungen der Formel Ia23, insbesondere die Verbindungen Ia23.1 bis Ia23.300, die sich von den Verbindungen Ia1.1 bis Ia1.300 dadurch unterscheiden, daß R¹ für Methoxy, R⁸ für Ethyl und R¹⁰ für Methyl stehen.

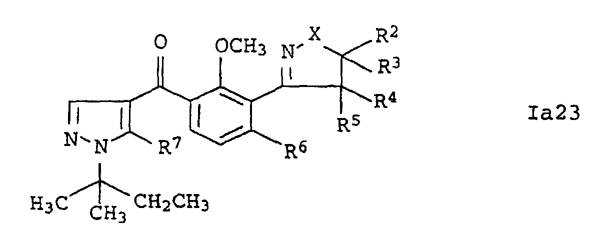

Ebenso außerordentlichst bevorzugt sind die Verbindungen der Formel Ia24, insbesondere die Verbindungen Ia24.1 bis Ia24.300, die sich von den Verbindungen Ia1.1 bis Ia1.300 dadurch unterscheiden, daß R¹ für Methoxy, R⁸ für Ethyl und R¹⁰ und R¹¹ für Methyl stehen.

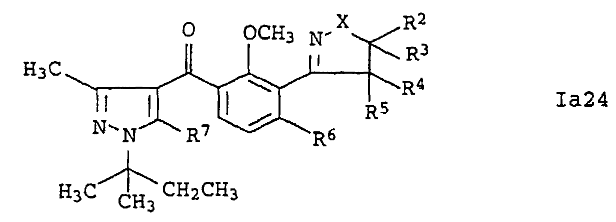

Die 3-(Heterocyclyl)-benzoylpyrazol-Derivate der Formel I, sind auf verschiedene Art und Weise erhältlich, beispielsweise nach folgenden Verfahren.

### Verfahren A:

Durch Umsetzung von Pyrazolen der Formel II mit einer aktivierten Benzoesäure IIIα oder einer Benzoesäure IIIβ, die vorzugsweise in situ aktiviert wird, zu dem entsprechenden Acylierungsprodukt IV und anschließende Umlagerung erhält man Verbindungen der Formel I mit R⁷ = OH.

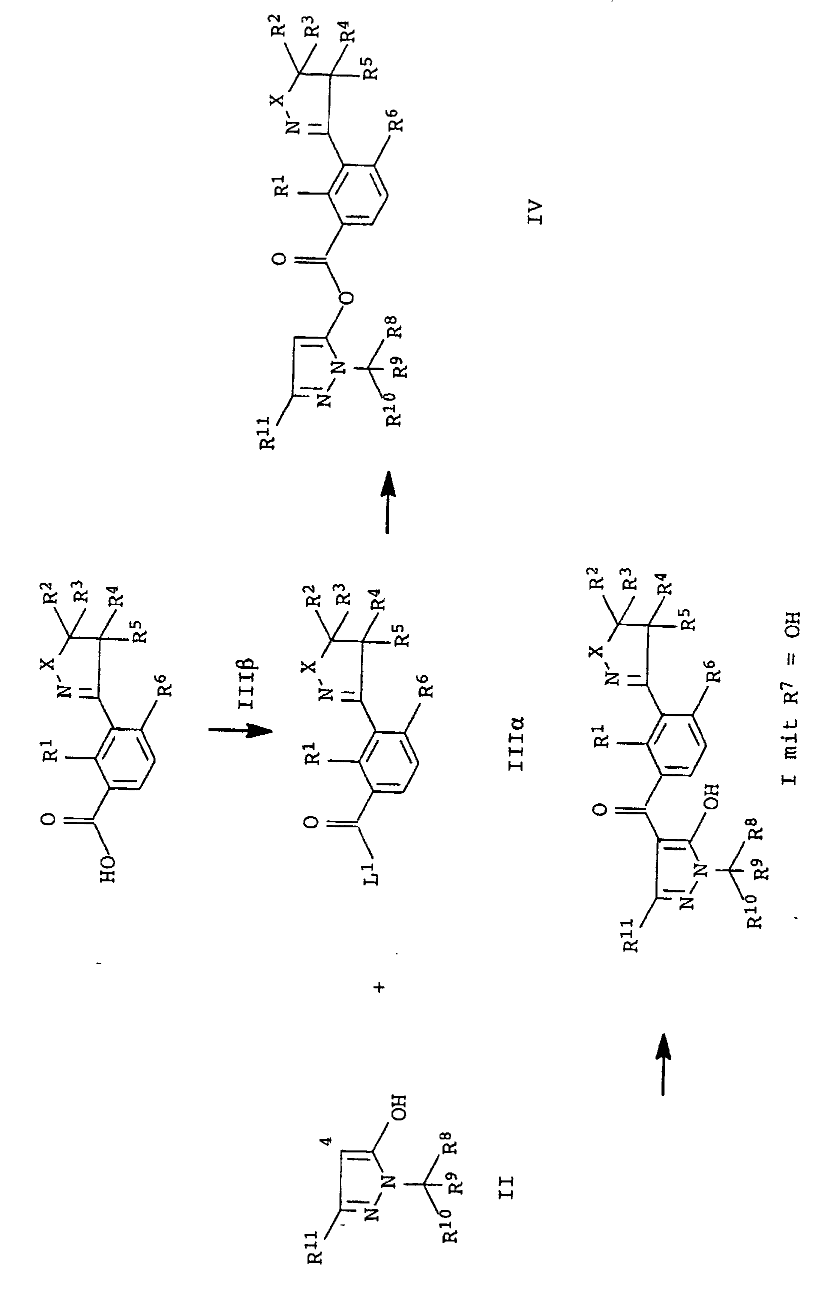

L¹ steht für eine nucleophil verdrängbare Abgangsgruppe, wie Halogen z.B. Brom, Chlor, Hetaryl, z.B. Imidazolyl, Pyridyl, Carboxylat, z.B. Acetat, Trifluoracetat etc.

Die aktivierte Benzoesäure kann direkt eingesetzt werden, wie im Fall der Benzoylhalogenide oder in situ erzeugt werden, z.B. mit Dicyclohexylcarbodiimid, Triphenylphosphin/Azodicarbonsäureester, 2-Pyridindisulfid/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid, 1,2-Dichlorethant, aromatische Kohlenwasserstoffe, wie Toluol, Xylol, Chlorbenzol, Ether, wie Diethylether, Methyl-tert.-butylether, Dimethoxyethan, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Benzoylhalogenide als aktivierte Carbonsäurekomponente eingesetzt, so kann es zweckmäßig sein, bei Zugabe dieses Reaktionspartners die Reaktionsmischung auf 0 - 10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether, Dimethoxyethan und Essigsäureethylester. Nach Trocknen der organischen Phase und Entfernen des Lösungsmittels kann der rohe Ester ohne weitere Reinigung zur Umlagerung eingesetzt werden.

Die Umlagerung der Ester zu den Verbindungen der Formel I erfolgt zweckmäßigerweise bei Temperaturen von 20 bis 40°C in einem Lösungsmittel und in Gegenwart einer Base sowie gegebenenfalls mit Hilfe einer Cyanoverbindung als Katalysator.

Als Lösungsmittel können z.B. Acetonitril, Methylenchlorid, 1,2-Dichlorethan, Dioxan, Essigsäureethylester, Dimethoxyethan, Toluol oder Gemische hiervon verwendet werden. Bevorzugte Lösungsmittel sind Acetonitril und Dioxan.

Geeignete Basen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonate verwendet, vorzugsweise in doppelt äquimolaren Verhältnis in Bezug auf den Ester.

Als Cyanoverbindungen kommen anorganische Cyanide, wie Natriumcyanid, Kaliumcyanid und organische Cyanoverbindungen, wie Acetoncyanhydrin, Trimethylsilycyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid, Essigsäureethylester extrahiert. Der organische Extrakt kann mit 5-10%iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt. (Beispiele für die Darstellung von Estern von Hydroxypyrazolen und für die Umlagerung der Ester sind z.B. in EP-A 282 944 und US 4 643 757 genannt).

Es ist aber auch möglich, das "Acylierungsprodukt" IV in situ zu erzeugen, indem man ein Pyrazol der Formel II, oder ein Alkalisalz hiervon, mit einem 3-(Heterocyclyl)-benzol-Derivat der Formel V in Gegenwart von Kohlenmonoxid, eines Katalysators sowie einer Base, umsetzt.

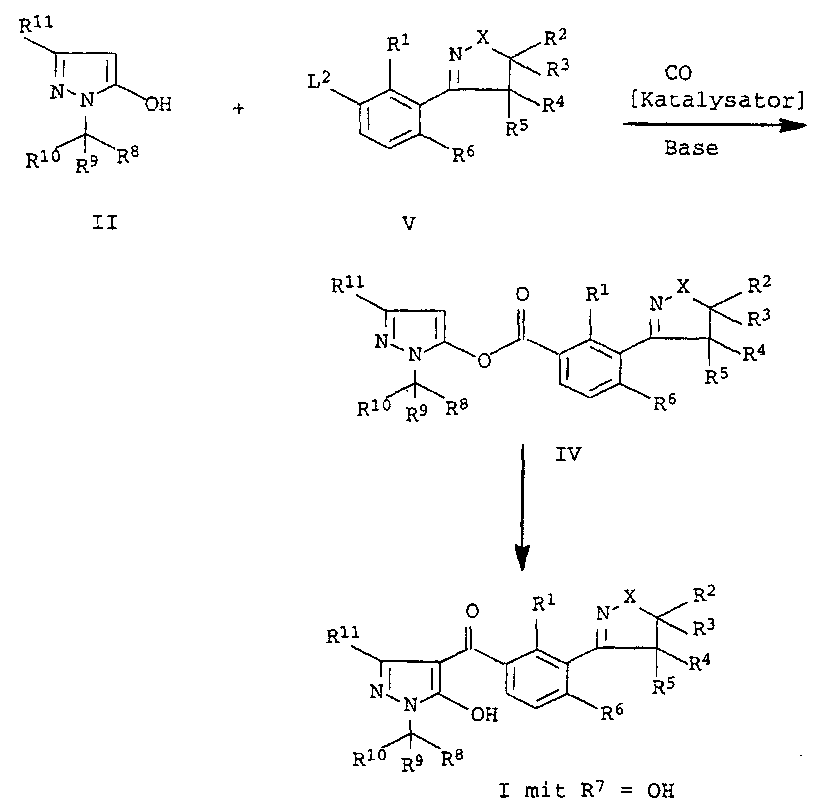

L² steht für eine Abgangsgruppe wie Halogen, z.B. Chlor, Brom oder Iod, oder Sulfonat wie Mesylat oder Triflat; bevorzugt sind Brom oder Triflat.

Das "Acylierungsprodukt" IV reagiert ggf. direkt zu dem 3-(Heterocyclyl)-benzoylpyrazol-Derivat der Formel I ab.

Als Katalysatoren eignen sich Palladiumligandkomplexe, in denen das Palladium in der Oxidationsstufe 0 vorliegt, metallisches Palladium, das gegebenenfalls auf einen Träger aufgezogen wurde, und vorzugsweise Palladium(II)salze. Die Umsetzung mit Palladium(II)salzen und metallischem Palladium wird vorzugsweise in Gegenwart von Komplexliganden durchgeführt.

Als Palladium (0) ligandkomplex kommt beispielsweise Tetrakis(triphenylphosphan)palladium in Frage.

Metallisches Palladium ist vorzugsweise auf einen inerten Träger wie beispielsweise Aktivkohle, Siliciumdioxid, Aluminiumoxid, Bariumsulfat oder Calciumcarbonat aufgezogen. Die Reaktion wird vorzugsweise in Gegenwart von Komplexliganden wie beispielsweise Triphenylphosphan durchgeführt.

Als Palladium (II) salze eigenen sich beispielsweise Palladiumacetat und Palladiumchlorid. Bevorzugt wird in Gegenwart von Komplexliganden wie beispielsweise Triphenylphosphan gearbeitet.

Geeignete Komplexliganden für die Palladiumligandkomplexe, bzw. in deren Gegenwart die Umsetzung mit metallischem Palladium oder Palladium (II) salzen vorzugsweise ausgeführt wird, sind tertiäre Phosphane, deren Struktur durch folgende Formeln wiedergegeben wird: 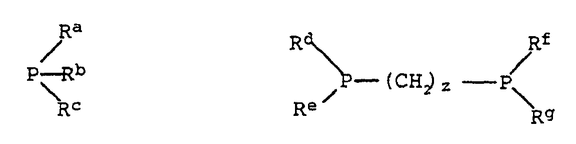 wobei z die Zahlen 1 bis 4 bedeutet und die Reste R^{a} bis R^{g} für C₁C₆-Alkyl, C₃-C₆-Cycloalkyl, Aryl-C₁-C₂-alkyl oder vorzugsweise Aryl stehen. Aryl steht beispielsweise für Naphthyl und gegebenenfalls substituiertes Phenyl wie beispielsweise 2-Tolyl und insbesondere für unsubstituiertes Phenyl.

Die Herstellung der komplexen Palladiumsalze kann in an sich bekannter Weise ausgehend von kommerziell erhältlichen Palladiumsalzen wie Palladiumchlorid oder Palladiumacetat und den entsprechenden Phosphanen wie z.B. Triphenylphosphan oder 1,2-Bis(diphenylphosphano)ethan erfolgen. Ein Großteil der komplexierten Palladiumsalze ist auch kommerziell erhältlich. Bevorzugte Palladiumsalze sind [(R)(+)2,2'-Bis(diphenylphosphano)-1,1'-binaphthyl]palladium(II)chlorid, Bis(triphenylphosphan)palladium(II)acetat und insbesondere Bis(triphenylphosphan)palladium(II)chlorid.

Der Palladiumkatalysator wird in der Regel in einer Konzentration von 0,05 bis 5 Mol%, bevorzugt 1-3 Mol%, eingesetzt.

Als Basen kommen tertiäre Amine wie beispielsweise N-Methylpiperidin, Ethyldiisopropylamin, 1,8-Bisdimethylaminonaphthalin oder insbesondere Triethylamin in Betracht. Ebenso eigenen sich Alkalicarbonate, wie Natriumcarbonat oder Kaliumcarbonat. Aber auch Gemische von Kaliumcarbonat und Triethylamin sind geeignet.

In der Regel werden 2 bis 4 Moläquivalente, insbesondere 2 Moläquivalente, des Alkalicarbonats, sowie 1 bis 4 Moläquivalente, insbesondere 2 Moläquivalente des tertiären Amins bezogen auf das 3-(Heterocyclyl)-benzol-Derivat der Formel V eingesetzt.

Als Lösungsmittel können Nitrile wie Benzonitril und Acetonitril, Amide wie Dimethylformamid, Dimethylacetamid, Tetra-C₁-C₄-alkylhamstoffe oder N-Methylpyrrolidon und vorzugsweise Ether wie Tetrahydrofuran, Methyl-tert.-butylether dienen. Insbesondere werden Ether wie 1,4-Dioxan und Dimethoxyethan als Lösungsmittel bevorzugt.

### Verfahren B:

Verbindungen der Formel I mit R⁷ ≠ Hydroxy werden durch Umsetzung von Verbindungen der Formel I mit R⁷ = Hydroxy mit Alkylierungsmitteln, Sulfonylierungsmittel bzw. Acylierungsmitteln L³-R^{7a} (VI) erhalten. 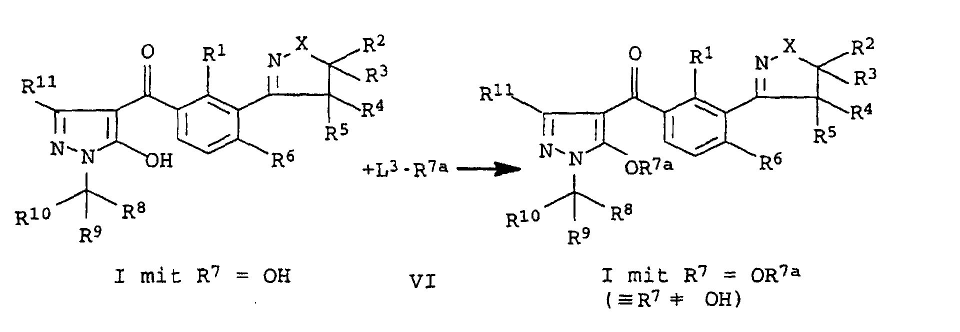
L³ steht für eine nucleophil verdrängbare Abgangsgruppe, wie Halogen, z.B. Brom oder Chlor, Acyloxy z.B. Acetyloxy, Ethylcarbonyloxy, oder Alkylsulfonyloxy, z.B. Methylsulfonyloxy oder Trifluormethylsulfonyloxy;
R^{7a} steht für C₁-C₆-Alkyl, C₃-C₆-Alkenyl, C₁-C₆-Alkylsulfonyl, C₁-C₆-Alkylcarbonyl, C₁-C₄-(Alkylthio)carbonyl, Phenylsulfonyl oder Phenylcarbonyl, wobei der Phenylrest der beiden letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy;

Die Verbindungen der Formel VI können direkt eingesetzt werden, wie z.B. im Fall der Sulfonsäurehalogenide, Sulfonsäureanhydride oder in situ erzeugt werden, z.B. aktivierte Sulfonsäuren (mittels Sulfonsäure und Dicyclohexylcarbonyldiimid, Carbonyldiimidazol etc.).

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuß der Hilfsbase z.B. 1,5 bis 3 Moläquivalente, bezogen auf I, kann unter Umständen vorteilhaft sein.

Als Hilfsbasen eignen sich tertiäre Alkylamine, wie Triethylamin, Pyridin, Alkalimetallcarbonate, z.B. Natriumcarbonat, Kaliumcarbonat und Alkalimetallhydride, z.B. Natriumhydrid. Bevorzugt verwendet werden Triethylamin und Pyridin.

Als Lösungsmittel kommen z.B. chlorierte Kohlenwasserstoffe, wie Methylenchlorid, 1,2 -Dichlorethan, aromatische Kohlenwasserstoffe, z.B. Toluol, Xylol, Chlorbenzol, Ether, wie Diethylether, Methyl-tert. -butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

Die als Ausgangsmaterialien verwendeten Pyrazole der Formel II sind bekannt oder können nach an sich bekannten Verfahren hergestellt werden (z.B. EP-A 240 001 und J. Prakt. Chem. 315, 383 (1973)).

Die aktivierten Benzoesäuren IIIα können auf an sich bekannte Art und Weise aus den Benzoesäuren IIIβ erhalten werden. Letztere werden wiederum durch Verseifung aus den entsprechenden Estern VII erhalten. Diese können dargestellt werden, indem man ein Oxim bzw. Hydrazon der Formel VIII in das entsprechende Hydroxyamsäurehalogenid, insbesondere Hydroxamsäurechlorid, bzw. Carbonsäurehydrazidhalogenid, insbesondere Carbonsäurehydrazidchlorid, überführt; in situ ein Nitriloxid bzw. Nitrilimin erzeugt und dieses mit einem Alken umsetzt (vgl. z.B. Chem. Ber. 106, 3258-3274 (1973).

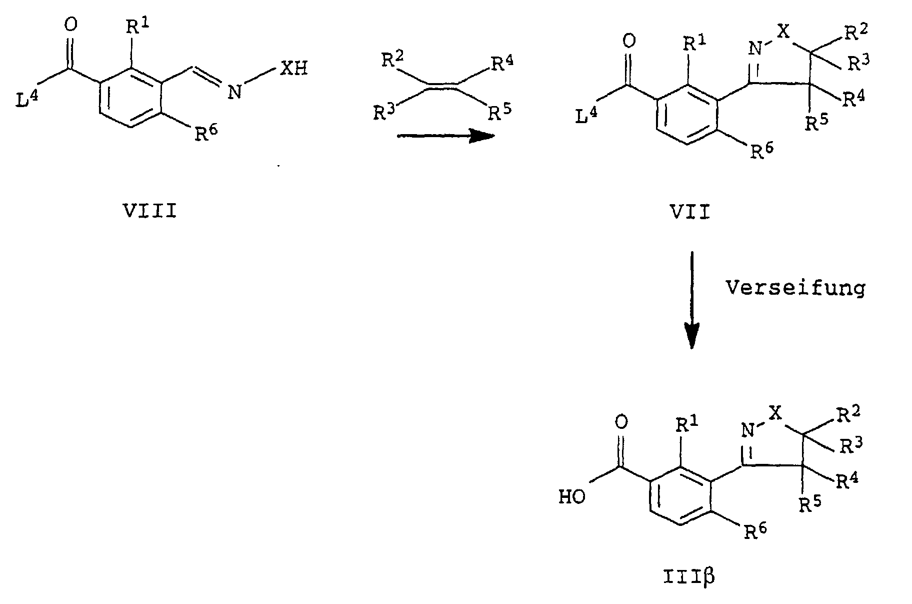

L⁴ steht für einen C₁-C₆-Alkoxyrest.

Die Benzoesäuren IIIβ können aber auch erhalten werden, indem man ein Oxim bzw. Hydrazon der Formel IX in die entsprechende Nitriloxide bzw. Nitrilimine überführt und diese mit Alkenen zu den entsprechenden Cycloadditionsprodukten umsetzt (vgl. z.B. Chem. Ber. 106, 3252-3274 (1973)). So wird beispielsweise das Oxim der Formel IX (X = O) mit Natriumhypochlorit oxidiert und mit einem geeigneten Alken in einem inerten Lösungsmittel, wie Methylenchlorid, Chloroform, Tetrahydrofuran, Dioxan oder Acetanitril, umgesetzt. Dieses wird dann in Gegenwart eines Katalysators sowie eine Base mit Kohlenmonoxid und Wasser in die Benzoesäure IIIβ überführt.

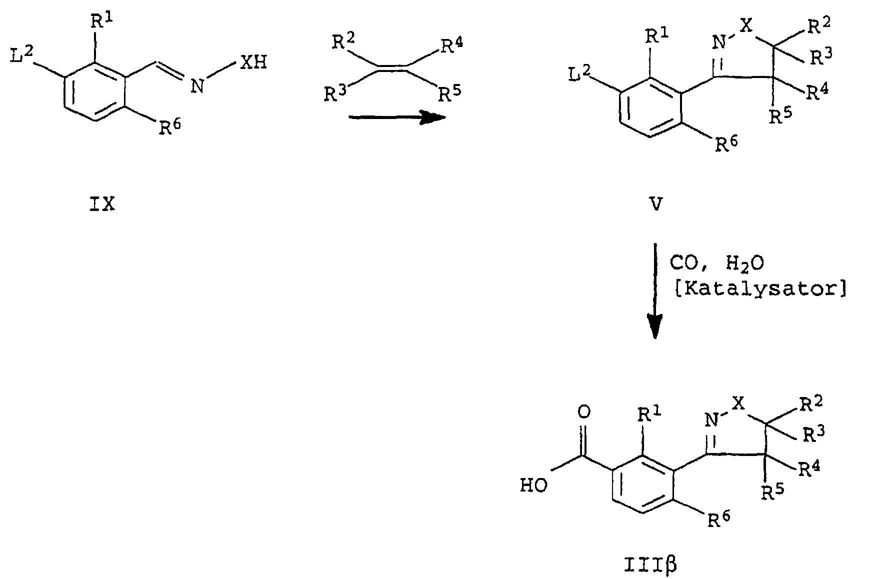

L² steht für eine Abgangsgruppe wie Halogen, z.B. Chlor, Brom oder Iod, oder Sulfonat wie Mesylat oder Triflat, bevorzugt sind Brom oder Triflat.

In Hinblick auf die Carbonylierungsreaktion gelten die voranstehenden Ausführungen in Analogie.

### Herstellungsbeispiele

### 4-[2-Chlor-3-(4,5-dihydroisoxazol-3-yl)-4-methylsulfonylbenzoyl]-5-hydroxy-1-(1,1-dimethyl-1-ethyl) -1H-pyrazol

### (Verbindung 2.1)

Zu einer Lösung von 5,4 g (0,02 mol) 2-Chlor-3-(4,5-dihydroisoxazol-3-yl)-4-methylsulfonylbenzoylchlorid in 100 ml Ethylenglykoldimethylether wurden 2,32 g (0,02 mol) 1-(1,1-Dimethyl-1-ethyl)5-hydroxy-1H-pyrazol und 2,3 g (0,02 mol) Kaliumcarbonat gegeben und über Nacht gerührt. Anschließend wurde 3 Stunden unter Rückfluß erhitzt, das Lösungsmittel abdestilliert, der Rückstand in 300 ml Wasser aufgenommen mit Methylenchlorid gewaschen und die wäßrige Phase mit 10 %iger Salzsäure angesäuert auf pH = 3. Der Niederschlag wurde abgesaugt und bei 40°C getrocknet. Man erhielt 4,6 g (65 % der Theorie).
4-[2-Chlor-3-(4,5-dihydroisoxazol-3-yl)-4-methylsulfonylbenzoyl]-5-hydroxy-1-(1,1-dimethyl-1-ethyl)-1H-pyrazol]

In Tabelle 2 sind neben der voranstehenden Verbindung noch weitere 3-(Heterocyclyl)-substituierte Benzoylpyrazole der Formel I aufgeführt, die in analoger Weise hergestellt wurden oder herstellbar sind.

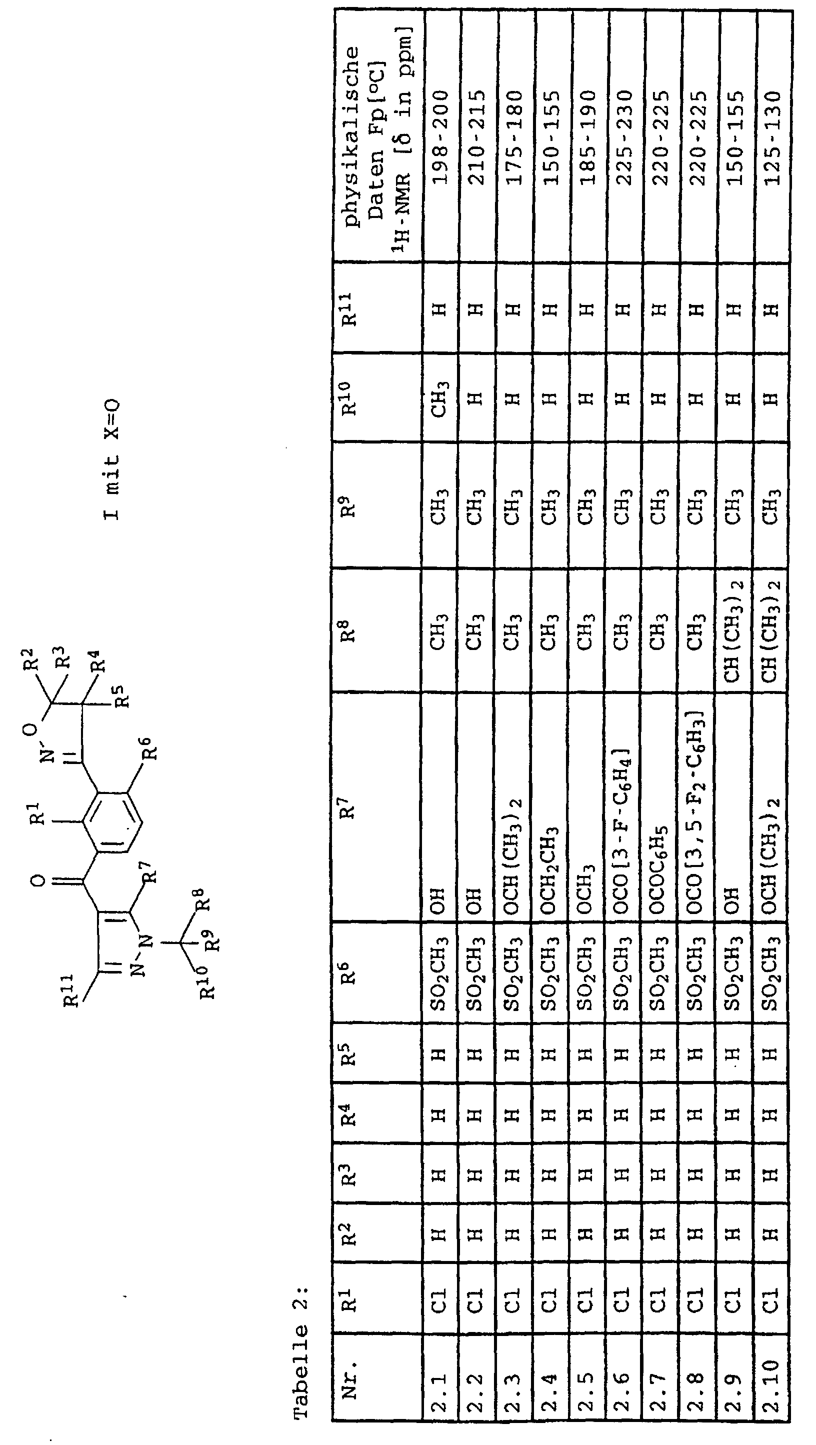

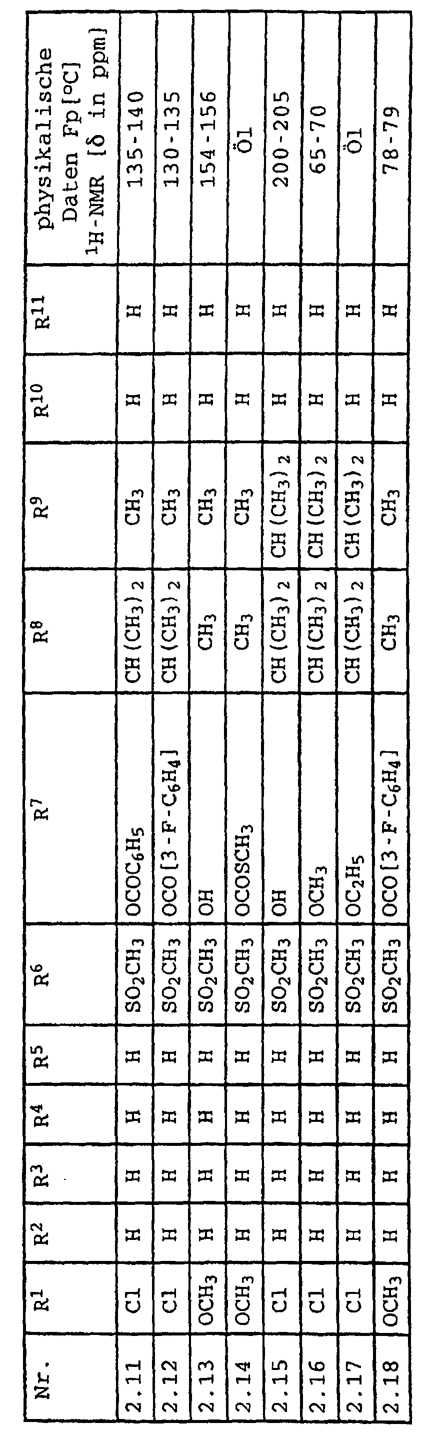

Die 3-(Heterocyclyl)-benzoylpyrazol-Derivate der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im Wesentlichen in Betracht:
Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. 2.2 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der-Verbindung Nr. 2.8 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. 2.8 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. 2.2 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile des Wirkstoffs Nr. 2.8 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 2.2 werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil des Wirkstoffs Nr. 2.8 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil des Wirkstoffs Nr. 2.2 wird in einer Mischung gelöst, die aus 80 Gewichtsteilen Cyclohexanon und 20 Gewichtsteilen Wettol® EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die 3-(Heterocyclyl)-benzoylpyrazolDerivate der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-{Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der 3-(Heterocyclyl)-benzoylpyrazol-Derivate der Formel I ließ sich durch die folgenden Gewächshausversuche zeigen:
Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,25 bzw. 0,125 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Chenopodium album | Weißer Gänsefuß | lamb's-quaters |
| Echinochloa crus-galli | Hühnerhirse | barnyard grass |
| Ipomoea ssp. | Prunkwindearten | morning glory |
| Polygonum persicaria | Flohknöterich | lady's-thumb |
| Setaria faberi | Große Borstenhirse | giant foxtail |
| Setaria viridis | Grüne Borstenhirse | green foxtail |
| Sinapis alba | Weißer Senf | white mustard |
| Solanum nigrum | Schwarzer Nachtschatten | black nightshade |

Bei Aufwandmengen von 0,25 bzw. 0,125 kg/ha zeigte die Verbindung 2.2 (Tabelle 2) im Nachauflauf eine sehr gute Wirkung sowohl gegen die Schadgräser Hühnerhirse und grüne Borstenhirse sowie den Unkräutern weißer Gänsefuß, Flohknöterich und schwarzer Nachtschatten. Weiterhin zeigte die Verbindung 2.8 (Tabelle 2) unter oben genannten Bedinungen sehr gute Wirkung gegen unerwünschte Pflanzen wie Hühnerhirse, große Borstenhirse, Prunkwindearten, weißer Senf und schwarzer Nachtschatten.

## Patentansprüche

1. 3-(Heterocyclyl)-benzoylpyrazol-Derivate der Formel I 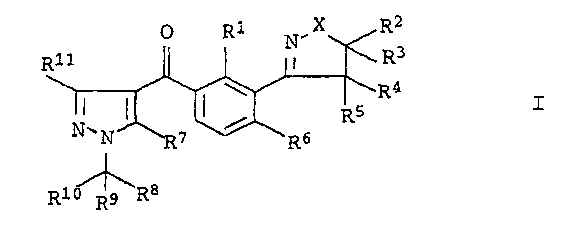 in der die Variablen folgende Bedeutung haben:
X O, NH oder N(C₁-C₆-Alkyl);
R¹ Nitro, Halogen, C₁-C₄-Alkoxy, C₁-C₄-Halogenalkoxy, C₁-C₄-Alkylthio, C₁-C₄-Halogenalkylthio, C₁-C₄-Alkylsulfonyl oder C₁-C₄-Halogenalkylsulfonyl;
R² ,R³ ,R⁴ ,R⁵ Wasserstoff, C₁-C₄-Alkyl oder C₁-C₄-Halogenalkyl;
R⁶ Halogen, Nitro, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfonyl oder C₁-C₄-Halogenalkylsulfonyl;
R⁷ Hydroxy, C₁-C₆-Alkoxy, C₃-C₆-Alkenyloxy, C₁-C₆-Alkylsulfonyloxy, C₁-C₆-Alkylcarbonyloxy, C₁-C₄-(Alkylthio)carbonyloxy, Phenylsulfonyloxy oder Phenylcarbonyloxy, wobei der Phenylrest der beiden letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy;
R⁸ ,R⁹ C₁-C₄-Alkyl;
R¹⁰ Wasserstoff oder C₁-C₄-Alkyl;
wobei die Anzahl der Kohlenstoffatome der Reste R⁸, R⁹ und R¹⁰ zusammen maximal 7 beträgt,
R¹¹ Wasserstoff oder C₁-C₄-Alkyl;
sowie deren landwirtschaftlich brauchbaren Salze.

2. 3-(Heterocyclyl)-benzoylpyrazol-Derivate der Formel I nach Anspruch 1, wobei
X O;
R¹ Nitro, Halogen, C₁-C₄-Alkoxy oder C₁-C₄-Alkylthio;
R⁶ C₁-C₄-Alkylthio oder C₁-C₄-Alkylsulfonyl;
bedeuten.

3. 3-(Heterocyclyl)-benzoylpyrazol-Derivate der Formel I nach Anspruch 1, wobei
X O;
R¹ Nitro, Halogen, C₁-C₄-Alkoxy oder C₁-C₄-Alkylthio;
R⁶ Halogen, Nitro, C₁-C₄-Halogenalkyl, C₁-C₄-Alkoxy oder C₁-C₄-Halogenalkoxy;
bedeuten.

4. 3-(Heterocyclyl)-benzoylpyrazol-Derivate der Formel I nach Anspruch 1, wobei
X N(C₁-C₆-Alkyl);
bedeutet.

5. Verfahren zur Darstellung von 3-(Heterocyclyl)-benzoylpyrazol-Derivaten der Formel I mit R⁷=Hydroxy, nach Anspruch 1, **dadurch gekennzeichnet, daß** man ein Pyrazol der Formel II 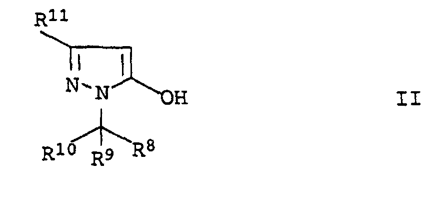 mit einer aktivierten Benzoesäure IIIα oder einer Benzoesäure IIIβ, 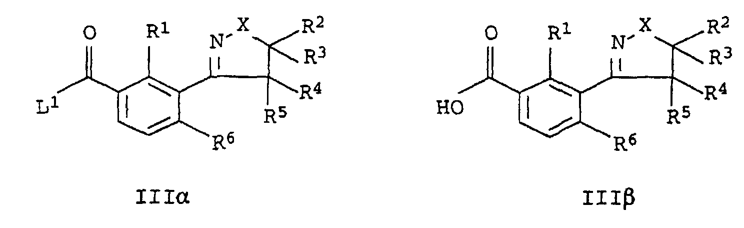 wobei die Variablen X, R¹ bis R⁶ und R⁸ bis R¹¹ die in Anspruch 1 genannte Bedeutung haben und L¹ für eine nucleophil verdrängbare Abgangsgruppe steht, acyliert und Acylierungsprodukt ggf. in Gegenwart eines Katalysators zu den Verbindungen der Formel I mit R⁷ = Hydroxy umlagert.

6. Verfahren zur Herstellung von 3-(Heterocyclyl)-benzoylpyrazol-Derivaten der Formel I mit R⁷ = OH, nach Anspruch 1, **dadurch gekennzeichnet, daß** man ein Pyrazol der Formel II, 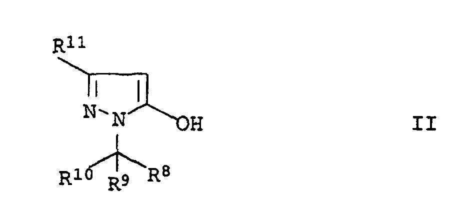 in der die Variablen R⁸ bis R¹¹ die in Anspruch 1 genannte Bedeutung haben, oder ein Alkalisalz hiervon, mit einem 3-(Heterocyclyl)-benzol-Derivat der Formel V, 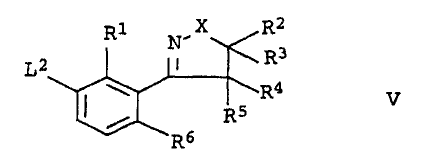 wobei die Variablen X und R¹ bis R⁶ die in Anspruch 1 genannte Bedeutung haben und L² für eine Abgangsgruppe steht, in Gegenwart von Kohlenmonoxid, eines Katalysators sowie einer Base umsetzt.

7. Verfahren zur Herstellung von 3-(Heterocyclyl)-benzoylpyrazol-Derivaten der Formel I mit R⁷ ≠ Hydroxy, nach Anspruch 1, **dadurch gekennzeichnet, daß** man ein 3-(Heterocyclyl)-benzoylpyrazol-Derivat I mit R⁷ = Hydroxy, 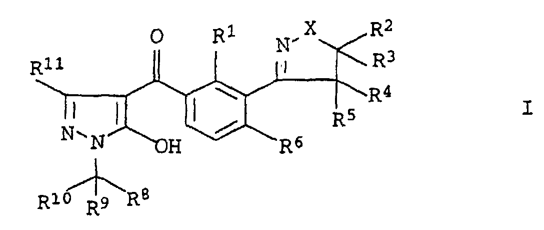 mit R⁷ = OH
mit einer Verbindung der Formel VI
L³-R^{7a} VI
umsetzt, wobei
L³ eine nucleophil verdrängbare Abgangsgruppe;
R^{7a} C₁-C₆-Alkyl, C₃-C₆-Alkenyl, C₁-C₆-Alkylsulfonyl, C₁-C₆-Alkylcarbonyl, C₁-C₄-(Alkylthio)carbonyl Phenylsulfonyl oder Phenylcarbonyl, wobei der Phenylrest der beiden letztgenannten Substituenten partiell oder vollständig halogeniert sein kann und/oder eine bis drei der folgenden Gruppen tragen kann:
Nitro, Cyano, C₁-C₄-Alkyl, C₁-C₄-Halogenalkyl, C₁-C₄-Alkoxy oder C₁-C₄-Halogenalkoxy;
bedeuten.

8. Mittel, enthaltend eine herbizid wirksame Menge mindestens eines 3-(Heterocyclyl)-benzoylpyrazol-Derivats der Formel I oder eines landwirtschaftlich brauchbaren Salzes von I gemäß den Ansprüchen 1 bis 4, und für die Formulierung von Pflanzenschutzmitteln übliche Hilfsmittel.

9. Verfahren zur Herstellung von Mitteln gemäß Anspruch 8, **dadurch gekennzeichnet, daß** man eine herbizid wirksame Menge mindestens eines 3-(Heterocyclyl)-benzoylpyrazol-Derivats der Formel I oder eines landwirtschaftlich brauchbaren Salzes von I gemäß den Ansprüchen 1 bis 4 und für die Formulierung von Pflanzenschutzmitteln übliche Hilfsmittel mischt.

10. Verfahren zur Bekämpfung von unerwünschtem Pflanzenwuchs, **dadurch gekennzeichnet, daß** man eine herbizid wirksame Menge mindestens eines 3-(Heterocyclyl)-benzoylpyrazol-Derivats der Formel I oder eines landwirtschaftlich brauchbaren Salzes von I gemäß den Ansprüchen 1 bis 4, auf Pflanzen, deren Lebensraum und/oder auf Samen einwirken läßt.

11. Verwendung der 3-(Heterocyclyl)-benzoylpyrazol-Derivate der Formel I und/oder deren landwirtschaftlich brauchbaren Salze gemäß den Ansprüchen 1 bis 4 als Herbizide.

## Claims

1. A 3-(heterocyclyl)benzoylpyrazole derivative of the formula I 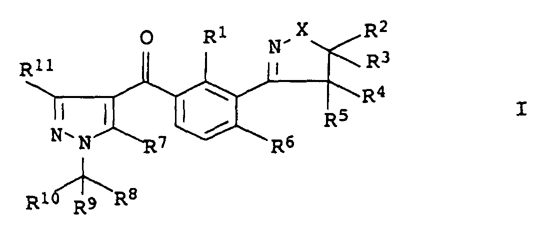 where
X is O, NH or N(C₁-C₆-alkyl);
R¹ is nitro, halogen, C₁-C₄-alkoxy, C₁-C₄-haloalkoxy, C₁-C₄-alkylthio, C₁-C₄-haloalkylthio, C₁-C₄-alkylsulfonyl or C₁-C₄-haloalkylsulfonyl;
R² ,R³ ,R⁴ ,R⁵ are hydrogen, C₁-C₄-alkyl or C₁-C₄-haloalkyl;
R⁶ is halogen, nitro, C₁-C₄-haloalkyl, C₁-C₄-alkoxy, C₁-C₄-haloalkoxy, C₁-C₄-alkylthio, C₁-C₄-haloalkylthio, C₁-C₄-alkylsulfonyl or C₁-C₄-haloalkylsulfonyl;
R⁷ is hydroxyl, C₁-C₆-alkoxy, C₃-C₆-alkenyloxy, C₁-C₆-alkylsulfonyloxy, C₁-C₆-alkylcarbonyloxy, C₁-C₄-(alkylthio)carbonyloxy, phenylsulfonyloxy or phenylcarbonyloxy, where the phenyl radical of the two last-mentioned substituents may be partially or fully halogenated and/or may carry one to three of the following groups:
nitro, cyano, C₁-C₄-alkyl, C₁-C₄-haioalkyl, C₁-C₄-alkoxy or C₁-C₄-haloalkoxy;
R⁸ ,R⁹ are C₁-C₄-alkyl;
R¹⁰ is hydrogen or C₁-C₄-alkyl;
where the number of the carbon atoms of the radicals R⁸, R⁹ and R¹⁰ together is at most 7,
R¹¹ is hydrogen or C₁-C₄-alkyl;
and its agriculturally useful salts.

2. A 3-(heterocyclyl)benzoylpyrazole derivative of the formula I as claimed in claim 1, where
X is O;
R¹ is nitro, halogen, C₁-C₄-alkoxy or C₁-C₄-alkylthio;
R⁶ is C₁-C₄-alkylthio or C₁-C₄-alkylsulfonyl.

3. A 3-(heterocyclyl)benzoylpyrazole derivative of the formula I as claimed in claim 1, where
X is O;
R¹ is nitro, halogen, C₁-C₄-alkoxy or C₁-C₄-alkylthio;
R⁶ is halogen, nitro, C₁-C₄-haloalkyl, C₁-C₄-alkoxy or C₁-C₄-haloalkoxy.

4. A 3-(heterocyclyl)benzoylpyrazole derivative of the formula I as claimed in claim 1, where
X is N(C₁-C₆-alkyl).

5. A process for preparing 3-(heterocyclyl)benzoylpyrazole derivatives of the formula I where R⁷=hydroxyl, as claimed in claim 1, which comprises acylating a pyrazole of the formula II 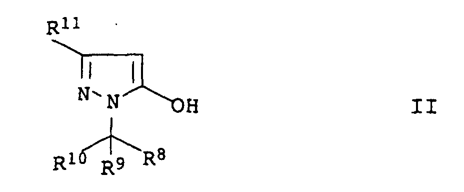 with an activated benzoic acid IIIα or a benzoic acid IIIβ, 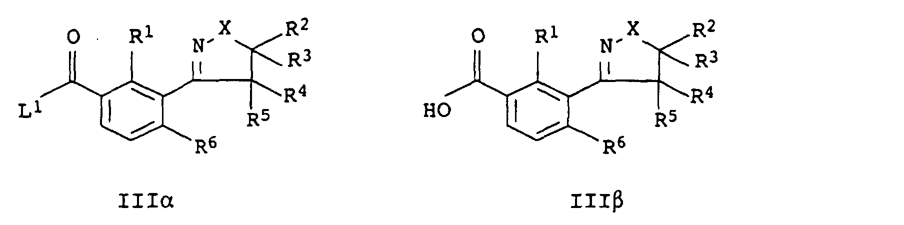 where the variables X, R¹ to R⁶ and R⁸ to R¹¹ are as defined in claim 1 and L¹ is a nucleophilically displaceable leaving group and rearranging acylation product, in the presence or absence of a catalyst, to give the compounds of the formula I where R⁷ = hydroxyl.

6. A process for preparing 3-(heterocyclyl)benzoylpyrazole derivatives of the formula I where R⁷ = OH, as claimed in claim 1, which comprises reacting a pyrazole of the formula II 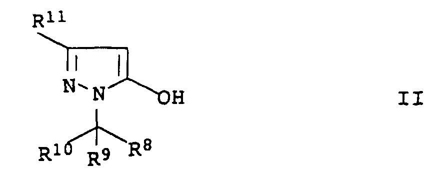 in which the variables R⁸ to R¹¹ are as defined in claim 1, or an alkali metal salt thereof, with a 3-(heterocyclyl)benzene derivative of the formula V 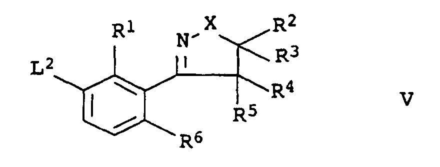 where the variables X and R¹ to R⁶ are as defined in claim 1 and L² is a leaving group, in the presence of carbon monoxide, a catalyst and a base.

7. A process for preparing 3-(heterocyclyl)benzoylpyrazole derivatives of the formula I where R⁷ ≠ hydroxyl, as claimed in claim 1, which comprises reacting a 3-(heterocyclyl)benzoylpyrazole derivative I where R⁷ = hydroxyl 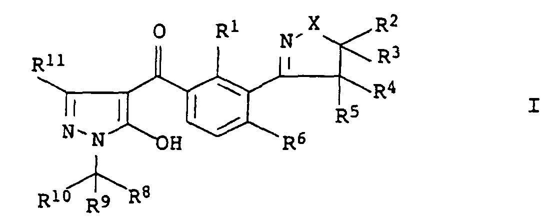 where R⁷ = OH
with a compound of the formula VI
L³-R^{7a} VI
where
L³ is a nucleophilically displaceable leaving group;
R^{7a} is C₁-C₆-alkyI, C₃-C₆-alkenyl, C₁-C₆-alkylsulfonyl, C₁-C₆-alkylcarbonyl, C₁-C₄-(alkylthio)carbonyl, phenylsulfonyl or phenylcarbonyl, where the phenyl radical of the two last-mentioned substituents may be partially or fully halogenated and/or may carry one to three of the following groups: nitro, cyano, C₁-C₄-alkyl, C₁-C₄-haloalkyl, C₁-C₄-alkoxy or C₁-C₄-haloalkoxy.

8. A composition, comprising a herbicidally effective amount of at least one 3-(heterocyclyl)benzoylpyrazole derivative of the formula I or an agriculturally useful salt of I, as claimed in any of claims 1 to 4, and auxiliaries which are customarily used for formulating crop protection agents.

9. A process for preparing compositions as claimed in claim 8, which comprises mixing a herbicidally effective amount of at least one 3-(heterocyclyl)benzoylpyrazole derivative of the formula I or an agriculturally useful salt of I, as claimed in any of claims 1 to 4, and auxiliaries which are customarily used for formulating crop protection agents.

10. A method for controlling undesirable vegetation, which comprises allowing a herbicidally effective amount of at least one 3-(heterocyclyl)benzoylpyrazole derivative of the formula I or an agriculturally useful salt of I, as claimed in any of claims 1 to 4 to act on the plants, their habitat and/or on seed.

11. The use of the 3-(heterocyclyl)benzoylpyrazole derivatives of the formula I and/or their agriculturally useful salts, as claimed in any of claims 1 to 4, as herbicides.

## Revendications

1. Dérivés de 3-(hétérocyclyl-benzoylpyrazoles répondant à la formule I 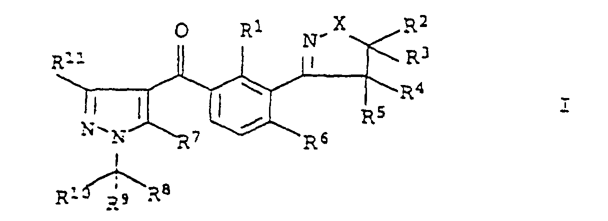 dans laquelle les symboles ont les significations suivantes :
X représente O, NH ou N-(alkyle en C1-C6);
R¹ représente un groupe nitro, un halogène, un groupe alcoxy en C1-C4, halogénoalcoxy en C1-C4, alkylthio en C1-C4, halogénoalkylthio en C1-C4, alkylsulfonyle en C1-C4 ou halogénoalkylsulfonyle en C1-C4;
R², R³, R⁴, R⁵ représentent l'hydrogène, des groupes alkyle en C1-C4 ou halogénoalkyle en C1-C4 ;
R⁶ représente un halogène, un groupe nitro, halogénoalkyle en C1-C4, alcoxy en C1-C4, halogénoalcoxy en C1-C4, alkylthio en C1-C4, halogénoalkylthio en C1-C4, alkylsulfonyle en C1-C4 ou halogénoalkylsulfonyle en C1-C4;
R⁷ représente un groupe hydroxy, alcoxy en C1-C6, alcényloxy en C3-C6, alkylsulfonyloxy en C1-C6, (alkyle en C1-C6)carbonyloxy, (alkylthio en C1-C4)carbonyloxy, phénylsulfonyloxy ou phénylcarbonyloxy, la partie phényle des deux substituants mentionnés en dernier pouvant être halogéné en totalité ou en partie et/ou pouvant porter un à trois des substituants suivants : nitro, cyano, alkyle en C1-C4, halogénoalkyle en C1-C4, alcoxy en C1-C4 ou halogénoalcoxy en C1-C4;
R⁸, R⁹ représentent des groupes alkyle en C1-C4;
R¹⁰ représente l'hydrogène ou un groupe alkyle en C1-C4;
le nombre des atomes de carbone des groupes R⁸, R⁹ et R¹⁰ étant au total de 7 au maximum,
R¹¹ représente l'hydrogène ou un groupe alkyle en C1-C4; et leurs sels aptes aux applications agricoles.

2. Dérivés de 3-(hétérocyclyl)-benzoylpyrazoles de formule I selon revendication 1 dans laquelle
X représente O;
R¹ représente un groupe nitro, un halogène, un groupe alcoxy en C1-C4 ou alkylthio en C1-C4;
R⁶ représente un groupe alkylthio en C1-C4 ou alkylsulfonyle en C1-C4.

3. Dérivés de 3-(hétérocyclyl)-benzoylpyrazoles de formule I selon revendication 1 dans laquelle
X représente O;
R¹ représente un groupe nitro, un halogène, un groupe alcoxy en C1-C4 ou alkylthio en C1-C4;
R⁶ représente un halogène, un groupe nitro, halogénoalkyle en C1-C4, alcoxy en C1-C4 ou halogénoalcoxy en C1-C4.

4. Dérivés de 3-(hétérocyclyl)-benzoylpyrazoles de formule I selon revendication 1 dans laquelle
X représente un groupe N-(alkyle en C1-C6).

5. Procédé pour la préparation des dérivés ue 3-(hétérocyclyl)-benzoyipyrazoles de formule I dans laquelle R7 représente un groupe hydroxy selon revendication 1, **caractérisé en ce que** l'on acyle un pyrazole de formule II 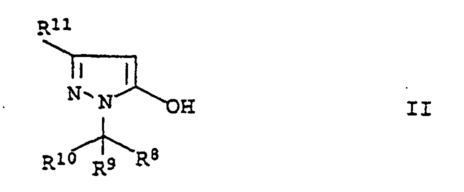 à l'aide d'un acide benzoïque activé IIIα ou d'un acide benzoïque IIIβ 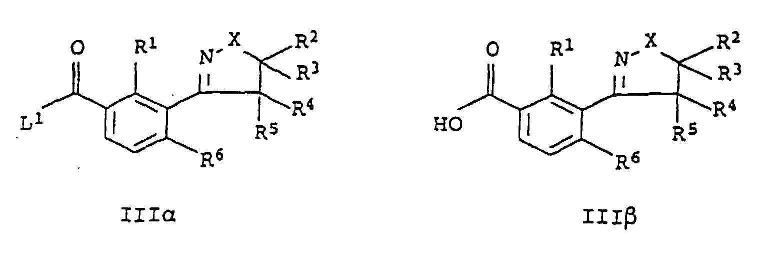 pour lesquels les symboles X, R¹ à R⁶ et R⁸ à R¹¹ ont les significations indiquées
dans la revendication 1 et L¹ représente un groupe éliminable par échange nucléophile, et on soumet le produit d'acylation à transposition, éventuellement en présence d'un catalyseur, en les composés de formule I dans laquelle R7 représente un groupe hydroxy.

6. Procédé pour la préparation des dérivés de 3-(hétérocyclyl)benzoylpyrazoles de formule I dans laquelle R⁷ = OH selon revendication 1, **caractérisé en ce que** l'on fait réagir un pyrazole de formule II 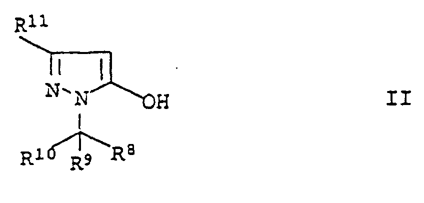 dans laquelle R⁸ à R¹¹ ont les significations indiquées dans la revendication 1, ou un sel alcalin de ce pyrazole, avec un dérivé de 3-(hétérocyclyl)-benzène de formule V 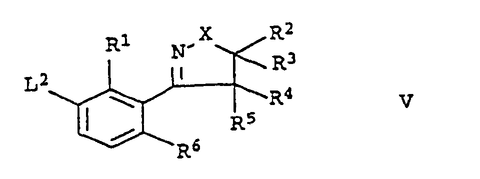 dans laquelle X et R¹ à R6 ont les significations indiquées dans la revendication 1 et L² représente un groupe éliminable, en présence de monoxyde de carbone, d'un catalyseur et d'une base.

7. Procédé pour la préparation des dérivés de 3-(hétérocyclyl)-benzoyipyrazoles de formule I dans laquelle R7 a une signification autre que le groupe hydroxy, selon revendication 1, **caractérisé par** la fait que l'on fait réagir un dérivé de 3-(hétérocyclyl)-benzoylpyrazole I pour lequel R⁷ représente un groupe hydroxy 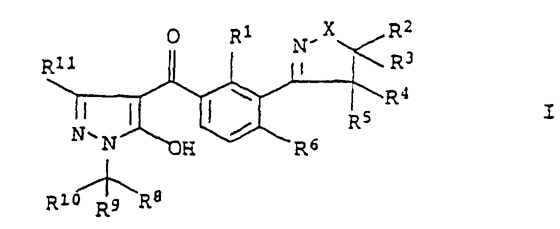 avec R⁷ = OH
avec un composé de formule VI
L³-R^{7a} VI
dans laquelle
L³ représente un groupe éliminable par échange nucléophile;
R 7^{a} représente un groupe alkyle en C1-C6, alcényle en C3-C6, alkylsulfonyle en C1-C6, (alkyle en C1-C6)carbonyle, (alkylthio en Cl-C4)carbonyle, phénylsulfonyle ou phénylcarbonyle, la partie phényle des deux substituants mentionnés en dernier pouvant être halogéné en totalité ou en partie et/ou pouvant porter un à trois des substituants suivants nitro, cyano, alkyle en C1-C4, halogénoalkyle en C1-C4, alcoxy en C1-C4 ou halogénoalcoxy en C1-C4.

8. Produit contenant une quantité herbicide efficace d'au moins un dérivé de 3-(hétérocyclyl)-benzoylpyrazole de formule I ou d'un sel de I apte aux applications agricoles selon les revendications 1 à 4 et des produits auxiliaires usuels pour la formulation de produits phytosanitaires.

9. Procédé pour la préparation de produits selon revendication 8, **caractérisé par le fait que** l'on mélange une quantité herbicide efficace d'au moins un dérivé de 3-(hétérocyclyl)-benzoylpyrazolet de formule I ou d'un sel de I apte aux applications agricoles selon les revendications 1 à 4, et des produits auxiliaires usuels pour la formulation de produits phytosanitaires.

10. Procédé pour combattre les croissances de végétaux indésirables, **caractérisé par le fait que** l'on fait agir une quantité herbicide efficace d'au moins un dérivé de 3-(hétérocyclyl)-benzoylpyrazole de formule I ou d'un sel de I apte aux applications agricoles selon les revendications 1 à 4 sur les végétaux, leur habitat et/ou sur les semences.

11. Utilisation des dérivés de 3-(hétérocyclyl)-benzoylpyraoles de formule I et/ou de leurs sels aptes aux applications agricoles selon les revendications 1 à 4 en tant qu'herbicides.
